# FASCICULE DE BREVET EUROPEEN

(11) **EP 0 368 920 B1**
(45) Date de publication et mention de la délivrance du brevet: **09.06.1993**
(21) Numéro de dépôt: 88907006.6
(22) Date de dépôt: 18.07.1988
(51) Int. Cl.: C07K 13/00, C07K 7/06, C07K 7/08, C07K 7/10, C07K 3/20, A61K 39/395, G01N 33/577, C07K 15/06, C07K 15/08

(54) **LECTINES FIXANT LE BETA-D-GALACTOSIDE**
LECTINE, DIE BETA-D-GALACTOSID BINDEN
LECTINES FIXING BETA-D-GALACTOSIDE

(30) Priorité: 21.07.1987 FR 8710288
(43) Date de publication de la demande: 23.05.1990
(73) Titulaire: INCYTE PHARMACEUTICALS, INC., Palo Alto , CA 94304 (US)
(72) Inventeur: STROSBERG, Arthur, Donny, F-75015 Paris (FR); TEICHBERG, Vivian, 76 100 Rehovot (IL)
(74) Mandataire: Ores, Irène
(86) Numéro de dépôt international: FR8800370
(87) Numéro de publication internationale: WO8900581

(56) Documents cités:
- 71st Annual Meeting of the Federation of American Societies for Experimental Biology, Washington, D.C., 29 March - 02 April 1987; Federation Proceedings, vol. 46, no. 3, A.D. Strosberg et al.: "Extensive structural homology between lactose binding lectins from fish, birds and mammals", p. 947
- Biological Abstracts, vol. 79, no. 1, 1985, abstract no. 622, Philadelphia, US; J. Hirabayashi et al.: "Human placenta beta-galactoside-binding lectin: Purification and some properties"
- Proc. Natl. Acad. Sci. USA, vol. 83, October 1986, The National Academy of Sciences (US), M.A. Gitt et al.: "Evidence that a human soluble beta-glactoside-binding lectin is encoded by a family of genes", pp. 7603-7607
- Chemical Abstracts, vol. 107, 1987 (Columbus Ohio, US) C. Southan et al.: "Amino acid sequence of beta-galactoside-binding bovine heart lectin. Member of a novel class of vertebrate proteins", p. 277 abstract no. 73017y

## Description

La présente invention est relative à l'isolement d'une lectine d'origine humaine, à la détermination de la séquence en amino-acides de lectines de provenances diverses, y compris des lectines d'origine humaine, et à leur synthèse, à l'application desdites séquences en thérapeutique en tant qu'immunostimulant et immunosuppresseur et en tant qu'agent interféron-like, pour le traitement de certaines viroses et certaines formes de cancer, à la production d'anticorps polyclonaux et monoclonaux anti-lectines et à leur application à l'immuno-diagnostic, à l'identification et l'isolement des gènes des lectines qui reconnaissent les séquences en amino-acides susdites, à l'utilisation de l'expression de ces gènes pour la production de lectines, de séquences en amino-acides et d'oligonucléotides propres à celles-ci.

Les lectines sont des protéines qui ont été trouvées dans une grande variété de vertébrés allant de l'anguille électrique (d'où leur nom) aux oiseaux et aux mammifères.

Les lectines sont des protéines qui fixent les sucres et se combinent avec des mono- et avec des oligosaccharides par des liaisons autres que co-valentes. Toutes les lectines sont des protéines oligomères comportant plusieurs sites de liaison de saccharides par molécule, c'est-à-dire qu'elles sont multivalentes. Cette structure polyvalente confère aux lectines l'aptitude d'agglutiner des cellules portant sur leur membrane externe les fractions saccharidiques appropriées.

Au cours des vingt dernières années, plusieurs centaines de lectines principalement extraites de plantes, ont été purifiées et caractérisées. Les lectines sont utilisées en tant que réactifs pour la purification et la caractérisation de glycoconjugués ou en tant que mitogènes de classes spécifiques de lymphocytes.

Actuellement, certaines lectines sont utilisées dans des banques de sang pour le typage du sang, principalement en raison de l'indisponibilité d'anticorps anti-O(H) naturels et en raison du fait que certaines d'entre elles distinguent les sous-groupes sanguins A₁ et A₂. Occasionnellement, les lectines sont utilisées pour la séparation de populations d'érythrocytes mixtes, par exemple dans les rares cas de mosaïques de groupes sanguins résultant de la formation de chimères, de mutations somatiques ou de transplantations de moëlle osseuse. D'autre part, comme certaines lectines [par exemple la phytohemagglutinine (PHA) et la concanavaline A] stimulent les lymphocytes B (par exemple le mitogène de phytolaque), les lectines sont également utilisées pour identifier les principales sous-populations de lymphocytes. Contrairement aux antigènes qui ne stimulent qu'une faible proportion des lymphocytes (habituellement de 0,02 à 0,2 %), les lectines stimulent une proportion élevée (jusqu'à 70-80 %) de cellules B, quelle que soit la spécificité antigénique des lymphocytes récepteurs. Les lectines sont donc des activateurs polyclonaux ou des ligands polyclonaux. Elles sont utilisées dans l'étude du mécanisme par lequel un antigène agissant à la surface de la cellule lymphoïde favorise spécifiquement ou inhibe spécifiquement l'expansion clonale et la synthèse des immunoglobulines. Comme autres produits synthétisés par des lymphocytes stimulés par des lectines, il faut citer diverses lymphokines, telles que l'interleukine 2 (I1-2) et l'interféron. La stimulation mitogénique, en particulier par la PHA, est également utilisée en tant qu'outil de diagnostic pour la détection de déficiences immunologiques congénitales et acquises, pour la détection de sensibilisations dues à des agents infectieux ou liées à certaines maladies auto-immunes, et pour le contrôle des effets de divers traitements immunosuppresseurs et immunothérapeutiques. Une autre application particulièrement utile de la stimulation des lymphocytes par ces lectines est représentée par les études cytogénétiques des chromosomes humains et animaux.

Les lectines sont également largement utilisées par les immunologistes pour étudier les propriétés et la constitution des membranes de lymphocytes. En utilisant des dérivés appropriés de lectines, il est possible d'examiner non seulement la répartition des récepteurs de lectines sur la surface des lymphocytes, mais également la mobilité des récepteurs dans la membrane, et de démontrer, par exemple, que la redistribution et le capping des récepteurs sur les surfaces des lymphocytes résultent de la fixation des lectines.

Les récepteurs de lectines, c'est-à-dire les constituants membranaires qui réagissent avec les lectines, peuvent être détectés sur des électrophorétogrammes ou être isolés sous une forme purifiée par les mêmes méthodes que celles qu'on utilise pour isoler des antigènes de surface à l'aide des anticorps correspondants. La détection est réalisée de préférence par coloration de la lectine dans les électrophorétogrammes de préparations de membranes. Les récepteurs peuvent être isolés à partir de membranes solubilisées, par précipitation soit par la lectine seule, soit en utilisant cette dernière associée à un anticorps antilectine ; les meilleurs résultats sont obtenus par isolement desdits récepteurs par chromatographie d'affinité sur des lectines immobilisées.

L'existence dans de nombreux tissus de vertébrés d'une activité lectine endogène fixant de façon spécifique le β-D-galactoside, a été démontrée en 1974 et attribuée à la famille de protéines dénommée "électrolectines" (en raison du fait que la première lectine animale décrite avait été isolée de l'organe électrique de l'anguille électrique - electrophorus electricus). Des lectines ont été isolées, depuis, de muscle, de rein, de coeur et d'intestin d'embryon de poulet, de coeur, de poumon et de rate de veau, de poumon de rat, de muscle de singe, de poumon et de muscle humain. De façon remarquable, les différentes lectines identifiées à ce jour présentent des propriétés très similaires à celles décrites pour l'électrolectine de l'anguille électrique. Toutes agglutinent les érythrocytes de lapin traités par la trypsine, toutes sont indiquées comme ayant principalement une localisation cytoplasmique intercellulaire, toutes ont un poids moléculaire compris entre 15 000 et 30 000 daltons et se composent de deux à quatre sous-unités, toutes possèdent la même affinité pour les saccharides suivants, dans un ordre décroissant :
β-D-galactosyl-thiogalactopyranoside (TDG) > lactose > D-galactose.

Toutes les lectines identifiées à ce jour ont été indiquées comme requérant la présence d'agents réducteurs pour le maintien de leur activité d'agglutination.

De plus, il a été montré que les lectines de divers tissus du même animal et que les lectines de tissus homologues de mammifères différents, présentent une réactivité antigénique croisée.

Les fonctions biologiques des lectines de vertébrés ne sont pas encore établies avec certitude. Il est cependant remarquable que leur concentration dans les tissus est régulée en fonction du développement et qu'elles sont associées à des processus néoplastiques. Chez l'animal adulte, les lectines de vertébrés sont principalement présentes dans des organes impliqués dans les défenses immunitaires (c'est-à-dire le thymus, le placenta, la peau). Les lectines présentant une spécificité vis-à-vis du lactose présentent des propriétés immunomodulatrices dans des maladies auto-immunes provoquées expérimentalement (telles que myasthenia gravis et diabète type I).

Les données actuellement disponibles indiquent que les lectines présentent une ressemblance structurale avec les interférons, qui sont des lymphokines déjà expérimentées en thérapie humaine pour certaines viroses et certaines formes de cancer. De plus, comme indiqué plus haut, l'étude fonctionnelle de l'électrolectine d'anguille a montré des propriétés immunosuppressives dans une forme expérimentale de myasthénie grave provoquée par l'injection de récepteur d'acétylcholine. Ces deux types d'observations suggèrent que les lectines pourraient être utilisées en clinique soit en tant que lymphokines, soit/et en tant que protéines immunosuppressives et en immunodiagnostic en utilisant la réaction de protéines antigéniques tumorales avec des anticorps antilectines.

Dans le but d'étudier la régulation de l'expression et les fonctions biologiques des lectines de vertébrés, GITT et BARONDES (PROC.NATL.ACAD.SCI.USA, Vol. 83, p. 7603-7607, Octobre 1986) ont visé à déterminer les séquences des lectines et plus particulièrement celle d'une lectine dimère humaine fixant le β-D-galactoside, dont la sous-unité présente un poids moléculaire de 14 000 daltons, cette lectine provenant de poumon humain. Ces Auteurs ont isolé deux clones de cDNA par immunocriblage d'une banque de cDNA d'hépatome humain, à l'aide d'un antisérum qui se lie spécifiquement à une lectine fixant le β-D-galactoside, de P.M. = 14 000 daltons. Ils ont trouvé que les séquences d'amino-acides des inserts de ces deux clones qu'ils ont ainsi déduites, présentent une homologie importante entre elles, avec la séquence de la lectine de peau de poulet, qui fixe le β-D-galactoside, et avec 8 peptides dérivés de lectine de poumon humain purifiée, de P.M. = 14 000 daltons. Cependant, des différences entre les séquences des deux clones d'hépatome et entre chacun de ces clones et les peptides de poumon humain, leur ont suggéré qu'il existerait au moins trois variantes du gène qui code pour cette lectine, qui seraient exprimées dans le tissu humain, étant cependant noté qu'ils n'ont pas établi la nature des protéines codées par les clones 1 et 2 d'hépatome humain.

Etant donné l'importance du rôle physiologique des lectines dans les évènements ontogéniques et les étapes de différenciation, il est apparu nécessaire d'établir l'homologie entre les diverses lectines de vertébrés en déterminant dans toute la mesure du possible leurs séquences, et d'élargir les sources disponibles de lectines homologues, notamment en vue de leur utilisation thérapeutique et pour le diagnostic.

C'est dans ces conditions que les Inventeurs ont déterminé l'homologie structurale de différentes lectines qui lient le β-D-galactoside, en établissant les séquences en amino-acides de ces lectines, isolées de l'anguille électrique et de placenta humain ; ils ont comparé les séquences qu'ils ont établies, avec des séquences publiées précédemment, déduites des cADN codant pour les lectines fixant le β-D-galactoside, isolées de peau d'embryon de poulet et d'hépatome humain, respectivement, et avec la séquence partielle en aminoacides de la lectine de poumon humain ; ils ont ainsi mis en lumière les homologies importantes entre ces lectines d'origines diverses.

La présente invention a pour objet une séquence d'amino-acides constituant un peptide, caractérisée en ce qu'elle correspond à une partie de la séquence des lectines fixant le β-D-galactoside, en ce que ledit peptide comprend un fragment qui contient un tryptophane ainsi que deux résidus d'acide glutamique, lequel fragment forme au moins en partie le site de fixation du β-D-galactoside et comprend la séquence (a) en amino-acides
Trp-Gly-Thr-Glu-Gln-Arg-Glu (a)
ou une séquence en amino-acides dans laquelle pas plus d'un des amino-acides Thr ou Gln, est remplacé par un autre amino-acide, à condition que ledit peptide ne contienne pas les séquences Gly-Ser-Asn, Met-Glu-Glu, Ala-Gly-Ala ou l'aminoacide Thr immédiatement en amont dudit fragment.

Conformément à la présente invention, le peptide correspondant à une partie de la séquence desdites lectines, comprend en outre la séquence :
X₁-X₁-Asn-X₁-Gly (b)
dans laquelle X₁ est un amino-acide hydrophobe choisi dans le groupe qui comprend Val, Ala, Met, Ile et Leu en amont de la séquence (a).

Selon une disposition avantageuse de l'invention, ladite séquence (b) est située en amont de la séquence (a), à une distance d'environ 35 résidus d'amino-acides.

Selon une autre disposition de l'invention, le peptide correspondant à une partie de la séquence desdites lectines, inclut en outre, en aval de ladite séquence (a), la séquence en amino-acides suivante :
Ile-Ile-Leu-Pro-Asp-Gly-X₂-X₂-X₂-X₂-Phe-Pro-Asn-Arg-Leu (c)
Selon une autre disposition avantageuse de l'invention, ladite séquence (c) est située en aval de la séquence (a), à une distance d'environ 30 résidus d'amino-acides.

Selon encore une autre disposition de l'invention, le peptide correspondant à une partie de la séquence desdites lectines, comprend, en outre, immédiatement en amont de ladite séquence (a), la séquence en amino-acides (d) suivante :
Asp-Gly-Gly-Ala (d)
Selon une autre disposition de l'invention, le peptide conforme à l'invention, inclut, en outre, immédiatement en aval de ladite séquence (a), la séquence en amino-acides (e) suivante :
Ala-Val-Phe-Pro-Phe-Gln-Pro-Gly-Ser-Val-Ala-Glu-Val (e)
Selon encore une autre disposition de l'invention, ledit peptide conforme à l'invention comprend un squelette d'amino-acides qui est composé d'au moins les amino-acides suivants, dans les positions suivantes :

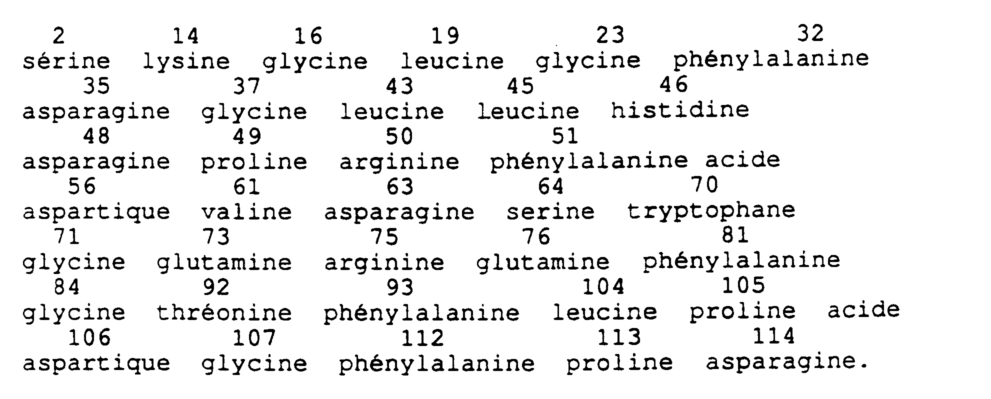

Selon une modalité de cette disposition, ledit peptide est caractérisé en ce que son squelette comprend en outre les amino-acides suivants dans les positions suivantes :

3          5              28              53        54
asparagine  valine  acide aspartique  alanine  histidine
55        67        68          72            74          80
glycine  glycine  glycine  thréonine  glutamine  proline
91            102          103            109
isoleucine  isoleucine  isoleucine  acide glutamique
132          133
isoleucine  lysine.

Selon une autre modalité de cette disposition, ledit peptide est caractérisé en ce que son squelette comprend en outre les amino-acides suivants, dans les positions suivantes : 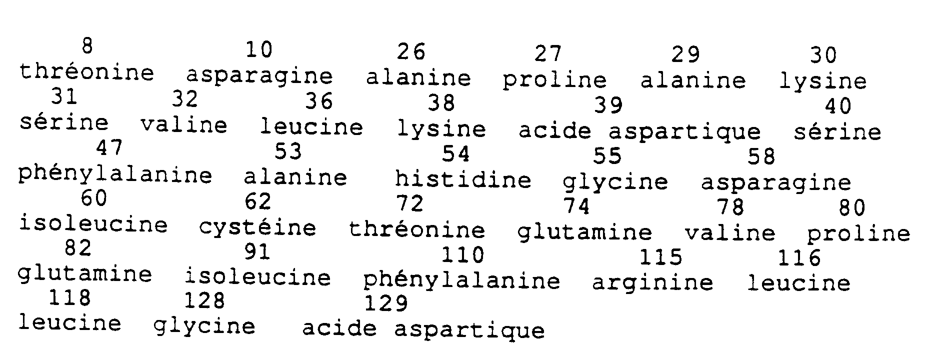
Selon encore une autre modalité de cette disposition, ledit peptide est caractérisé en ce que son squelette comprend en outre les amino-acides suivants, dans les positions suivantes :

4 6 18 20 21 22
glycine valine glutamine thréonine valine lysine
52 53 54 55 72
acide aspartique alanine histidine glycine thréonine
74 80 91 96
glutamine proline isoleucine asparagine.

Selon une autre modalité de cette disposition, ledit peptide est caractérisé en ce que son squelette comprend en outre les amino-acides suivants, dans les positions suivantes :

        28                67          68          74
acide aspartique  glycine  glycine  glutamine
        79
phénylalanine.

Selon une autre modalité de cette dispostion, ledit peptide est caractérisé en ce que son squelette comprend en outre les amino-acides suivants, dans les positions suivantes :

26 27 29 30 31 33 36
alanine proline alanine lysine sérine valine leucine
38 39 40 74 78
lysine acide aspartique sérine glutamine valine
79 109 110 115 116
phénylalanine glutamine phénylalanine arginine leucine
118 123
leucine tyrosine.

Selon encore une autre disposition de l'invention, la séquence d'amino-acides constituant un peptide est caractérisée en ce qu'elle comprend au moins les 129 amino-acides suivants, qui sont présents selon la même séquence dans la lectine de l'anguille électrique :

2                                   10
(SER MET) ASN GLY VAL VAL ASP GLU ARG MET SER PHE LYS ALA
                20                                      30
GLY GLN ASN LEU THR VAL LYS GLY VAL PRO SER ILE ASP SER THR
                                        40
ASN PHE ALA ILE ASN VAL GLY ASN SER ALA GLU ASP LEU ALA LEU
                50                                      60
HIS ILE ASN PRO ARG PHE ASP ALA HIS GLY ASP GLN GLN ALA VAL
                                         70
VAL VAL ASN SER PHE GLN GLY GLY ASN (TRP) GLY (THR) GLU
                           80
GLN (ARG) GLU GLY GLY PHE PRO PHE LYS GLN GLY GLU ASP PHE
        90                                      100
LYS ILE GLN ILE THR PHE ASN SER GLU GLU PHE ARG ILE ILE LEU
                            110
PRO ASP GLY SER GLU ILE HIS PHE PRO ASN ASN ARG TYR MET HIS
        120
PHE GLU GLY GLU ALA ARG ILE TYR SER ILE GLU ILE LYS...

Selon encore une autre disposition avantageuse de l'invention, la séquence d'amino-acides est caractérisée en ce qu'elle comprend au moins les amino-acides suivants, qui se présentent selon la même séquence dans la lectine de placenta humain : 
 1 4 6 8 10 11
SER ( ) ASN TYR VAL SER ( ) THR ( ) ASN
18 20 21 23 26 28
( ) ILE GLY GLU VAL ALA PRO ASP
30 31 34 35 36 40 41 42 43
ALA LYS SER PHE VAL LEU ASN LEU GLY LYS ASP SER ASN ASN LEU
45 46 48 50 51 53 54 55 56
CYS LEU HIS PHE ASN PRO ARG PHE ASN ALA HIS GLY ASP ALA ASN
60 61 67 68 70 71 72
THR ILE VAL LYS ASN SER ( ) ASP GLY GLY ALA TRP GLY THR
74 78 80 81 82 83 84 85 86 87
GLU GLN ARG GLU ALA VAL PHE PRO PHE GLN PRO GLY SER VAL ALA
88 89 90 91 92 93 94 95 96 97 100
GLU VAL ( ) ILE THR PHE ASP GLN ALA ASN LEU LEU VAL
101 102 103 104 105 107 108 109 110 111 112 113 114 115
ILE ILE LEU PRO ASP GLY LEU GLU PHE LYS PHE PRO ASN ARG
116 118 119 120 121 123 125 128 130
LEU ASN LEU GLU ALA ILE ASN LEU MET ALA ALA ASP GLY ASP PHE
131 132 133 134 135
LYS ILE LYS
Les séquences d'amino-acides conformes à la présente invention correspondent à une partie de la séquence de lectines qui fixent le β-D-galactoside, présentent une affinité plus grande pour le β-D-galactosyl-thiogalactopyranoside que pour le lactose et plus grande pour ce dernier que pour le galactose, et requièrent des agents réducteurs pour maintenir leur activité agglutinante ; ces séquences comprennent des déterminants essentiels desdites lectines et présentent une réactivité croisée à l'égard d'anticorps dirigés contre des lectines d'origines diverses, tant humaines qu'animales.

Conformément à la présente invention, les amino-acides de la séquence qui reproduit celle de la lectine de l'anguille électrique entrent dans la composition de plusieurs peptides qui ont été analysés par dégradation, dans un micro-séquenceur en phase gazeuse, et dont les amino-acides qui les composent ont été identifiés par chromatographie haute pression en phase liquide (HPLC), les peptides suivants ayant ainsi été identifiés :

(1) Asn - Ser - Glu - Glu - Phe - Arg

(2)  Ala - Gly - Gln - Asn - Leu - Thr - Val
(3)  Phe - Asn - Ser - Glu - Glu - Phe - Arg
(4)  Tyr - Met - His - Phe - Glu - Gly - Glu - Ala - Arg
(5)  Glu - Gly - Gly - Phe - Pro
(6)  Ile - Tyr - Ser - Ile - Glu - Ile
(7)  Ile - Tyr - Ser - Ile - Glu - Ile - Lys
(8)  Phe - Asp - Ala - His - Gly - Asp - Gln - Gln - Ala -
     Val - Val - Val - Asn - Ser - Phe - Gln - Gln - Asn
(9)  Ile - Gln - Ile - Thr - Phe - Asn - Ser - Glu - Glu -
     Phe - Arg
(10) Ile - Ile - Leu - Pro - Asp - Gly - Ser - Glu - Ile -
     His - Phe - Pro - Asn - Asn - Arg
(11) Gly - Val - Pro - Ser - Ile - Asp - Ser - Thr - Asn -
     Phe - Ala - Ile - Asn - Val - Gly - Asn - Ser - Ala
     Glu - Asp - Leu - Ala - Leu - His - Ile - Asn - Pro -
     Arg.

Conformément à l'invention, les séquences d'amino-acides conformes à la présente invention qui correspondent à une partie de la séquence des lectines telles que définies plus haut, présentent une structure en feuillets plissés β, lesdits feuillets plissés étant au moins au nombre de 10, dans des positions correspondant à des positions d'amino-acides communes aux lectines de diverses origines.

Les séquences d'amino-acides conformes à la présente invention se distinguent, en outre, par leur caractère hydrophobe, établi à l'aide de l'échelle d'hydropathie de KYTE ET DOOLITTLE.

Les séquences d'amino-acides conformes à la présente invention se distinguent également par le fait que le tryptophane qu'elles contiennent se trouve dans une position comprise entre la position 69 et la position 76 de la séquence, ainsi que deux résidus acide glutamique, et en ce que la région 69-76 forme au moins en partie le site de fixation du β-D-galactoside.

Lesdites séquences d'amino-acides se distinguent d'autre part par le fait qu'elles contiennent au moins deux résidus cystéine ou demi-cystine qui se trouvent en positions 44 et 62 et dont l'état chimique devrait jouer un rôle important dans l'intégrité fonctionnelle de la protéine.

Les séquences d'amino-acides conformes à la présente invention se distinguent en outre par le fait qu'elles comportent un peptide terminal bloqué sur l'azote, qui se compose d'une séquence Ser-Met N-acétylée.

La présente invention a également pour objet un procédé de purification de lectines fixant le β-D-galactoside à partir de tissus d'animaux vertébrés et notamment de l'anguille électrique et d'organes humains, tels que le placenta notamment, par homogénéisation et fractionnement des tissus animaux, puis isolement par chromatographie d'affinité à l'aide d'une matrice de lactosyl-Sépharose, lequel procédé est caractérisé en ce que le tampon d'élution utilisé pour isoler les lectines est constitué par une solution saline pH 7,2, tamponnée par du phosphate, additionnée de lactose et de 2-mercaptoéthanol.

La présente invention a, de plus, pour objet un procédé de détermination des séquences - ou séquençage - d'amino-acides correspondant à une partie de la séquence des lectines fixant le β-D-galactoside définies plus haut, qui est caractérisé par la mise en oeuvre d'une méthode de dégradation automatique par couplage de phénylisothiocyanate avec l'azote terminal du peptide à séquencer, clivage dudit résidu amino-terminal par cyclisation en milieu acide, et conversion du dérivé de thiazolinom formé, en un dérivé de phénylthiohydantoïne, laquelle méthode de dégradation est réalisée en présence de polybrène.

La présente invention a également pour objet un procédé d'obtention par voie de synthèse de séquences d'amino-acides correspondant à une partie de la séquence de lectines fixant le β-D-galactoside, caractérisé en ce que ladite synthèse est réalisée en mettant en oeuvre une méthode dérivée de la méthode de BERGMAN et ZERVAS et utilisant des groupes protecteurs pour protéger les fonctions réactives des amino-acides à coupler, et des méthodes de couplage appropriées pour coupler entre eux les amino-acides protégés, pour former les séquences recherchées.

La présente invention a, en outre, pour objet des anticorps polyclonaux anti-lectine placentaire humaine, caractérisés en ce qu'ils sont essentiellement constitués par des sérums obtenus à partir de sang d'animaux immunisés par injection de lectine telle que définie ci-dessus, purifiée par le procédé de purification des lectines tel que défini ci-dessus.

La présente invention a également pour objet des anticorps monoclonaux anti-lectine placentaire humaine, caractérisés ence qu'ils sont constitués par des clones isolés à partir d'hybridomes obtenus par fusion de cellules tumorales appropriées, SP 2-0 ou NS-1 notamment, avec des splénocytes de souris immunisées contre la lectine de placenta humain telle que définie ci-dessus, convenablement purifiée notamment à l'aide du procédé de purification tel que défini ci-dessus.

La présente invention a également pour objet un agent de diagnostic d'affections tumorales, caractérisé en ce qu'il comprend des anticorps polyclonaux et/ou des anticorps monoclonaux tels que définis ci-dessus.

La présente invention, de plus, pour objet un agent thérapeutique caractérisé en ce qu'il comprend une lectine comprenant la séquence (a) en amino-acides et immédiatement en aval de ladite séquence (a), la séquence en aminoacides (e), éventuellement associée à au moins un véhicule pharmaceutiquement acceptable.

La présente invention a, en outre, pour objet un agent thérapeutique, caractérisé en ce qu'il comprend des anticorps polyclonaux et/ou des anticorps monoclonaux tels que définis ci-dessus, éventuellement associés à des toxines et/ou des enzymes.

Outre les dispositions qui précèdent, l'invention comprend encore d'autre dispositions qui ressortiront de la description qui va suivre, qui comprend des exemples d'obtention des séquences d'amino-acides définies dans ce qui précède et des méthodes de contrôle de l'activité de lectines fixant le β-D-galactoside, de ces séquences, en référence aux dessins annexés dans lesquels :
- la Figure 1 représente la séquence en acides aminés de la lectine d'anguille électrique, faisant apparaître l'alignement des peptides et des fragments qui la composent ;
- la Figure 2 représente un profil HPLC typique d'une digestion de l'électrolectine par la trypsine, dans lequel chacune des fractions obtenues par digestion a été analysée dans un séquenceur en phase gazeuse et dans lequel la Séquence correspondante est indiquée au-dessus du pic ;
- la Figure 3 représente une comparaison des séquences obtenues respectivement pour les lectines fixant le β-D-galactoside, isolées d'anguille électrique, de placenta humain (Hum.p.), et de poumon humain (Hum. 1.) avec celles obtenues à partir des cDNA de peau d'embryon de poulet et d'hépatome humain Li-7 ("Hum.hep.1", "Hum.hep.2") :
la Figure 4 représente les structures en feuillets plissés β et les profils d'hydropathie des lectines fixant le β-D-galactoside isolées de poulet et d'anguille électrique. Les deux courbes du haut ont été calculées à l'aide de la méthode de CHOU & FASMAN [(BIOCHEMISTRY, 13, (1974), 211-222] ; les deux courbes du bas ont été calculées en utilisant l'échelle d'hydropathie de KYTE & DOOLITTLE [J. MOL. BIOL. 157 (1982) 105-132].
la figure 5 représente la migration de la lectine humaine en gel de polyacrylamide, obtenue par électrofocalisation ;
la figure 7 représente les espèces moléculaires identifiées par hybridation, par la méthode de SOUTHERN, et

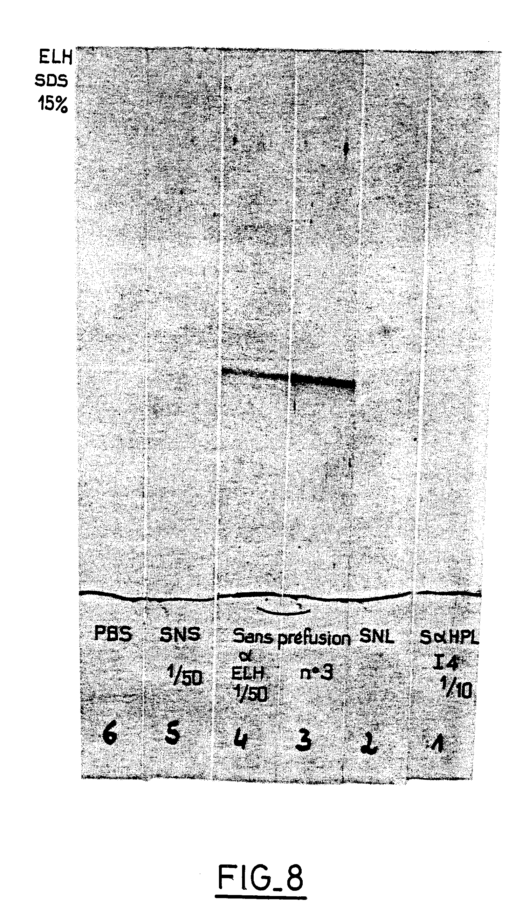

la figure 8 représente un immunoblot de migration de lectine purifiée.

Il doit être bien entendu, toutefois, que ces exemples sont donnés uniquement à titre d'illustration de l'objet de l'invention, dont ils ne constituent en aucune manière une limitation.

### EXAMPLE I - Purification de lectine fixant le β-D-galactoside, à partir d'anguille électrique

Des anguilles électriques, Electrophorus electricus, (obtenues vivantes du WORLDWIDE PARAMOUNT AQUARIUM/ARDSLEY, N.Y.) ont été décapitées et leur principal organe électrique a été découpé en petits cubes et congelé à -20°C.

L'homogénéisation des tissus et le fractionnement ont été ralisés comme décrit par LEVI & TEICHBERG, J. BIOL. CHEM., 256 (1981), 5735-5740. Les lectines ont été isolées par chromatographie d'affinité sur une matrice de lactosyl-Sepharose, comme décrit dans cette dernière Publication. Le tampon d'élution était constitué par une solution saline, pH 7,2, tamponnée par 0,01 M de phosphate et additionnée de 100 mM de lactose et 14 mM de 2-mercapto-éthanol.

La pureté de la lectine éluée a été vérifiée par électrophorèse sur polyacrylamide-dodécylsulfate de sodium et son activité après dialyse contre une solution saline tamponnée au phosphate, a été contrôlée sur des érythrocytes de lapins trypsinisés dans un test quantitatif d'hémogglutination effectué sur des plaques de microtitration comme décrit également dans la Publication précitée.

### EXAMPLE II - Purification de lectine fixant le β-D-galactoside à partir de placenta humain

Des placentas humains frais provenant d'accouchements nocturnes ont été maintenus sur de la glace jusqu'à leur collecte et leur traitement, dès le matin suivant.

La lectine a été récupérée en procédant comme décrit à l'Exemple I.

La pureté et l'homogénéité de la lectine placentaire humaine ont été vérifiées par électrophorèse en polyacrylamide en présence d'un gradient de pH (technique dite d'"isofocalisation") suivant la procédure suivante :
Le pHi de la lectine humaine (ELH) est déterminé par isoélectrofocalisation (IEF), en procédant comme suit :

### Technique

### Gel polyacrylamide solution stock

24,25 g acrylamide.
0,75 g bis acrylamide.
2,50 g amberlite MB-6 (Pharmacia).
250,00 ml eau distillée.

### Préparation du gel (plaques 115 x 230 mm)

Filtrer 15 ml de la solution stock.
1,90 ml Pharmalyte (Gradient pH 3.10).
4,00 ml glycérol.
Ajuster à 30 ml avec H₂0.
Dégazer.
Ajouter 300 µl N,N,N',N'-tétraméthyl-éthylendiamin (TEMED).
Couler le gel.
Laisser polymériser une heure.

### Migration :

Placer le gel sur le plateau réfrigérant de l'appareil.
Disposer les électrodes de chaque côté, bandelette de papier LKB imprégnée d'une solution H₃PO₄, 1 M pour l'anode et NaOH 1 M pour la cathode.
Préfocalisation.
20 minutes à puissance constante 40 mW.
Dépôt des échantillons (à 1 cm de l'anode).
Sur papier LKB de 1 x 0,5 cm.
ELH : 20 µl déposé d'une solution à 600 µg/ml soit 12 µg.
Protéines témoin : 20 µl solution étalon Pharmacia.
Migration : 1h30, puissance 40 mW constante.

### Révélation :

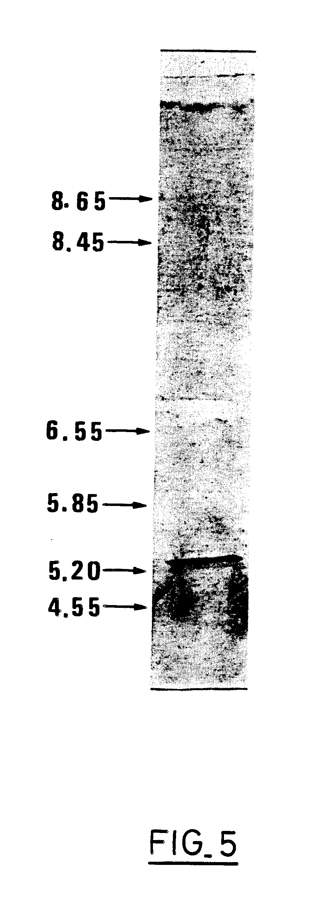

Fixation 45 minutes acide trichloracétique 10 % puis 30 minutes dans une solution 30 % éthanol 5 % acide acétique.
Coloration 1 heure au bleu de Coomassie.
Décoloration dans éthanol 40 % + 5 % acide acétique.
Séchage.
On trace alors la courbe pHi = f (distance de migration) (cm) pour les protéines étalon.
Le pHi de la lectine humaine ainsi déterminé est de 5,25, comme le montre la figure 5 annexée.

### EXEMPLE III - Séparation et purification des peptides

### 1. Digestions enzymatiques.

a) La digestion par la trypsine a été réalisée dans du NH₄HCO₃ 1 % par addition de 10 µg de trypsine traitée par de la tosylphénylalanylchlorométhylcétone, dissoute dans 0,01 mM d'HCl, à 1 mg de lectine isolée d'anguille électrique ou de placenta humain, comme décrit aux Exemples I et II ci-dessus. Au bout de 6 heures de digestion enzymatique à 37°C, 10 µg supplémentaires de trypsine ont été ajoutés et la digestion a été arrêtée 6 heures plus tard en abaissant le pH jusqu'à 4. Le produit de digestion a été séparé par HPLC sur une colonne RP 300 en utilisant le gradient suivant :
   - solvant A :: H₂O/0,1 % TFA
   - solvant B :: CH₃CN 80 %/H₂O 20 %/0,1 % TFA
   (TFA = Acide trifluoroacétique).
   Les deux solvants ont été dégazés par passage d'un courant d'hélium. La séparation a été menée à son terme en utilisant un gradient de 0 % de solvant B à 40 % de solvant B en l'espace de 8 heures, à un débit de 0,5 ml/minute. Le détecteur à longueur d'ondes variable a été réglé à 206 nm avec une sensibilité de 0,2 nm. La température de l'opération était de 25°C.
b) La digestion par la protéase de Staphylococcus aureus (décrite dans HOUMARD & DRAPEAU, PROC. NATL. ACAD. SCI., 69, 3506-3509) a été réalisée dans des conditions propres à réaliser le clivage au niveau des liaisons peptidiques Glu-X : 10 µg de l'enzyme (fournie par MILES LABORATORIES) ont été ajoutés à 1 mg de lectine dans 50 mM de NH₄HCO₃ à pH 7,8 et la digestion s'est poursuivie pendant 18 heures à 37°C. Les peptides résultants ont été séparés par HPLC comme décrit plus haut.

### 2. Clivage par le bromure de cyanogène.

Ce clivage a été réalisé dans de l'acide formique à 70 %, à 25°C pendant 24 heures à l'obscurité, en utilisant 10 mg de bromure de cyanogène pour 1 mg de lectine.

Les fragments résultants ont été séparés par gelfiltration sur du Sephadex G-50 Superfine, en présence de chlorhydrate de guanidine 5M. Les fragments ont été relargués sur du Sephadex G-25 dans du NH₄HCO₃ à 1 % et lyophilisés.

Les peptides obtenus par digestion par la trypsine et par la protéase de Staphylococcus aureus, ont été séparés par chromatographie en phase inverse sur un instrument de WATERS, et contrôlés à 206 nm à l'aide d'un spectrophotomètre 440. La plupart des séparations ont été réalisées sur une colonne RP 300 (fournie par BROWNLEE) dans des tampons d'acide trifluoroacétique (TFA) à 1 %, en utilisant les gradients décrits plus haut.

### EXEMPLE IV - Séquençage des amino-acides contenus dans les peptides de lectine.

### 1. Analyse de la séquence.

Les différents peptides ont été analysés par dégradation d'EDMAN automatisée, dans un microséquenceur en phase gazeuse (commercialisé par APPLIED BIOSYSTEMS). Du polybrène traité chimiquement (vendu sous la marque "CHEMUBRENE" par CHEMUNEX, PARIS) a été ajouté pour empêcher l'extraction des peptides et pour améliorer les rendements. Les amino-acides de la phénylthiohydantoïne ont été identifiés par HPLC sur un instrument de WATERS équipé d'une colonne RP 18 de 5µ de diamètre (fournie par BROWNLEE) en utilisant un gradient d'acétate de sodium/acétonitrile.

### a) Analyse des fragments de lectines clivés par le bromure de cyanogène.

La composition en amino-acides de la lectine d'anguille indique la présence de trois résidus méthionine. Pour tirer avantage de la présence de ces résidus, la protéine a été hydrolysée en utilisant du bromure de cyanogène dans de l'acide formique à 70 %. On n'a isolé qu'un seul long fragment et un petit fragment, qui ont été purifiés et partiellementment séquencés. Le troisième peptide à azote terminal (bloqué) n'a pas été récupéré.

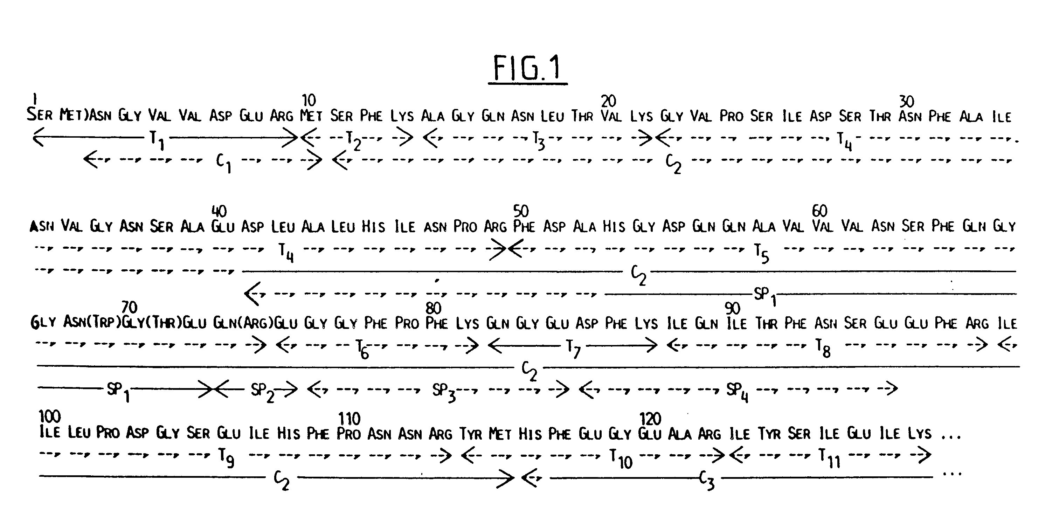

Les résultats de ces clivages sont résumés dans la Figure 1 annexée qui représente l'alignement des peptides et des fragments de lectine isolée d'anguille électrique ; dans cette Figure, C₁, C₂ et C₃ désignent les fragments obtenus par clivage par le bromure de cyanogène. En remplaçant l'acide formique à 70 % par une solution 50:50 HFBA/acide formique (HFBA = acide heptafluorobutyrique), on a observé un clivage en un site supplémentaire, qui correspond selon toute probabilité à une liaison peptidique tryptophane -X (X: amino-acide non identifié). Ce nouveau peptide a été partiellement séquencé pour obtenir la séquence Gly-(Thr)-Glu-Gln.

### b) Analyse des peptides résultant de la digestion par la trypsine.

La majeure partie de la séquence de la lectine d'anguille électrique et la totalité des résultats relatifs à la protéine humaine ont été obtenus par analyse des peptides obtenus par digestion par la trypsine, séparés sur une colonne RP 300 comme décrit au paragraphe 1.a) du présent Exemple.

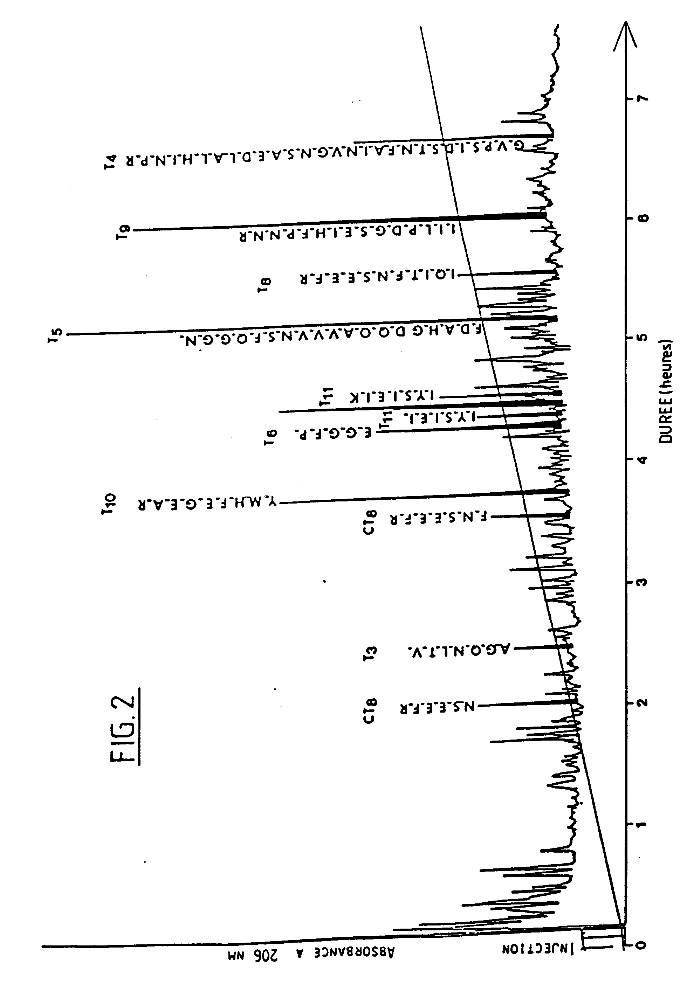

La Figure 2 annexée représente une séparation typique obtenue sur colonne RP 300 ; les séquences des peptides correspondants sont indiquées le long des pics, avec les significations suivantes :
- A: Ala Alanine
- B: Asx Asp ou Asn
- C: Cys Cystéine ou demi-cystine
- D: Asp Acide aspartique
- E: Glu Acide glutamique
- F: Phe Phénylalanine
- G: Gly Glycine
- H: His Histidine
- I: Ile Isoleucine
- K: Lys lysine
- L: Leu Leucine
- M: Met Méthionine
- N: Asn Asparagine
- O: PCA Gln cyclisé
- P: Pro Proline
- Q: Gln Glutamine
- R: Arg Arginine
- S: Ser Sérine
- T: Thr Thréonine
- V: Val Valine
- W: Trp Tryptophane
- X: ( ) inconnu
- Y: Tyr Tyrosine
- Z: Glx Glu ou Gln
Les pics ont été numérotés en fonction de la position des peptides dans la totalité de la séquence représentée dans la Figure 1, dans laquelle lesdits peptides sont désignés par T₁, T₂, T₃, T₄, T₅, T₆, T₇, T₈, T₉, T₁₀, T₁₁ (T = digestion par la trypsine). Deux peptides supplémentaires (CT 8) ont été obtenus, probablement par action de la chymotrypsine contenue dans la trypsine.

Les séquences indiquées à chacun des pics de la Figure 2 sont les suivantes :

CT8:N.S.E.E.F.R=Asn.Ser.Glu.Glu.Arg.
T3:A.G.Q.N.L.T.V.=Ala.Gly.Gln.Asn.Leu.Thr.Val.
CT8:F.N.S.E.E.F.R.=Phe.Asn.Ser.Glu.Glu.Phe.Arg.
T10:Y.M.H.F.E.G.E.A.R.=Tyr.Met.His.Phe.Glu.Gly
Glu.Ala.Arg.
T6:E.G.G.F.P.=Glu.Gly.Gly.Phe.Pro
T11:I.Y.S.I.E.I.=Ile.Tyr.Ser.Ile.Glu.Ile
T11:I.Y.S.I.E.I.K.=Ile.Tyr.Ser.Ile.Glu.Ile.Lys.
T5:F.D.A.H.G.D.Q.Q.A.V.V.V.N.S.F.Q.G.G.N=Phe.Asp.
Ala.His.Gly.Asp.Gln.Gln.Ala.Val.Val.Val.Asn.
Ser.Phe.Gln.Gly.Gly.Asn.
T8:I.Q.I.T.F.N.S.E.E.F.R.=Ile.Gln.Ile.Thr.Phe.Asn.
Ser.Glu.Glu.Phe.Arg.
T9:I.I.L.P.D.G.S.E.I.H.F.P.N.N.R.=Ile.Ile.Leu.Pro.
Asp.Gly.Ser.Glu.Ile.His.Phe.Pro.Asn.Asn.Arg.
T4:G.V.P.S.I.D.S.T.N.F.A.I.N.V.G.N.S.A.E.D.L.A.L.
H.I.N.P.R.=Gly.Val.Pro.Ser.Ile.Asp.Ser.Thr.Asn.
Phe.Ala.Ile.Asn.Val.Gly.Asn.Ser.Ala.Glu.Asp.
Leu.Ala.Leu.His.Ile.Asn.Pro.Arg.

### c) Analyse des peptides résultant de la digestion par la protéase de Staphylococcus aureus.

Des séquences supplémentaires de lectine et des sé quences en chevauchement ont été obtenues par analyse des peptides résultant de la digestion de lectine d'anguille électrique par la protéase extraite de Staphylococcus aureus qui, dans les conditions expérimentales mises en oeuvre, clive les protéines aux liaisons peptidiques Glu-X. Les peptides ont été séparés par HPLC avant d'analyser leur séquence à l'aide du séquenceur automatisé. Les peptides clivés par la protéase de Staphylococcus aureus sont désignés à la Figure 1 par SP₁, SP₂, SP₃, SP₄.

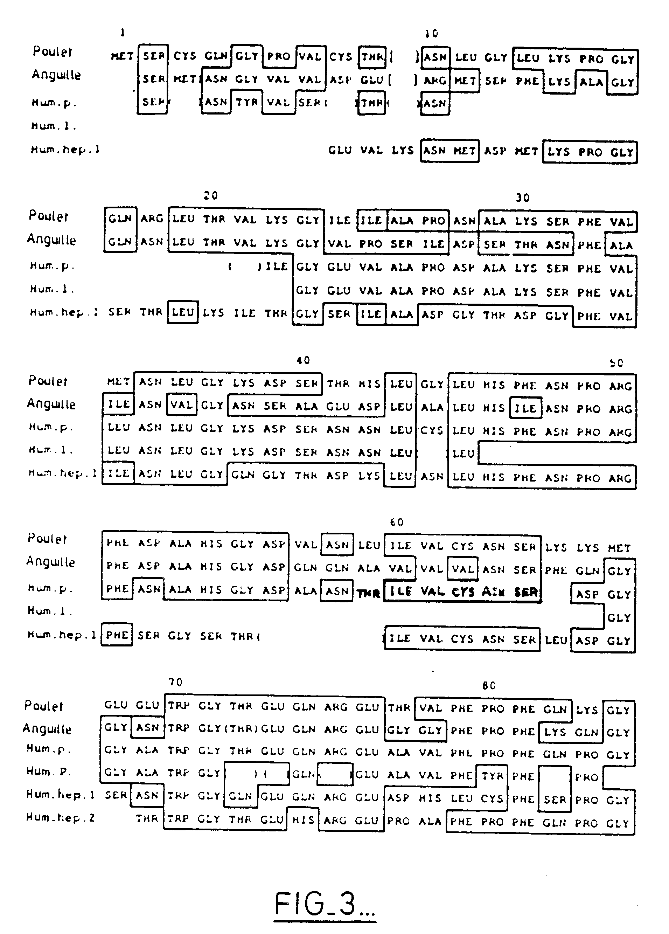

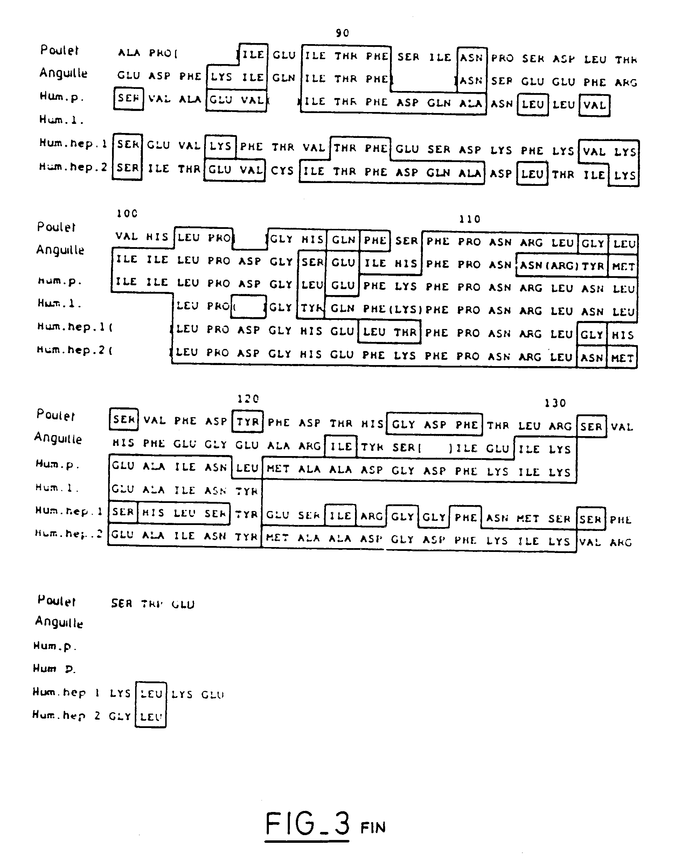

Les analyses des séquences des peptides obtenus aussi bien par clivage chimique que par clivages protéolytiques, ont permis d'établir la totalité de la séquence en amino-acides de la lectine fixant le β-D-galactoside, isolée de l'anguille électrique et plus de 60 % de celle de la lectine placentaire humaine. L'alignement des peptides tel qu'il est représenté à la Figure 3 annexée, pour la lectine de poulet, d'anguille électrique, de placenta humain (Hum.p.), de poumon humain (Hum.1.) et d'hépatome humain (Hum. Hep. 1 et 2), est basé sur les homologies importantes que présentent les autres lectines avec la protéine de 14KD qui fixe le β-D-galactoside, isolée d'embryon de poulet.

### 2. Structures secondaires des lectines

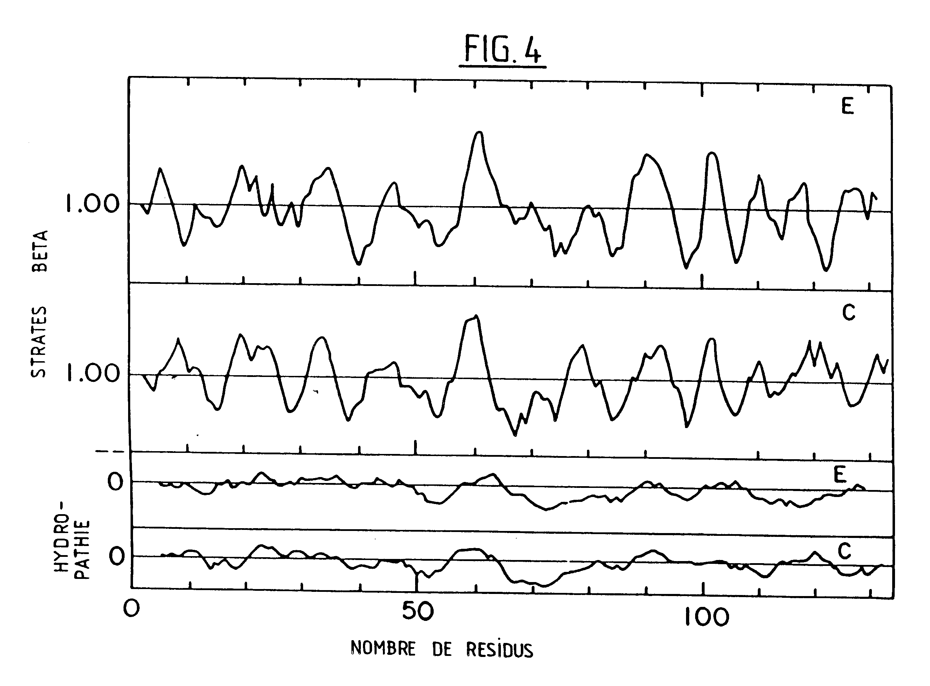

Les paramètres caractéristiques des structures secondaires des lectines ont été calculés sur des lectines de poulet et d'anguille, à l'aide d'un logiciel fourni par le groupe d'informatique appliquée au génie génétique, de l'Université de Wisconsin, et d'un ordinateur "Microwax II". Les résultats de ces calculs sont représentés à la Figure 4 annexée qui représente la propension des lectines à former des structures en feuillets plissés β et qui représente également leurs profils d'hydropathie. Les deux courbes supérieures - qui représentent les structures en feuillets plissés β en fonction du nombre de résidus d'amino-acides - ont été calculées à l'aide de la méthode de CHOU & FASMAN [BIOCHEMISTRY, 13, 211-222] et les deux courbes inférieures - qui représentent l'hydropathie, en fonction du nombre de résidus d'amino-acides - ont été obtenues à l'aide de l'échelle d'hydropathie de KYTE & DOOLITTLE [J. MOL. BIOL., 157, 105-132].

Dans la Figure 4, les parties de chacune des courbes qui se trouvent au-dessus de la ligne de seuil dont la valeur est de 1,00, indiquent les résidus d'amino-acides probablement impliqués dans les structures en feuillets plissés β. Il s'ensuit que les lectines de poulet et d'anguille semblent être toutes deux composées d'au moins 10 feuillets plissés β qui sont tous constitués par des résidus d'amino-acides dans des positions équivalentes dans les deux protéines.

Il en est de même des profils d'hydropathie dont la similarité est très grande dans les deux protéines.

Les séquences d'amino-acides qui ont pu être établies, de même que la propension des lectines à former des feuillets plissés β et leurs profils d'hydropathie, tels que représentés à la Fig. 4, mettent en évidence l'homologie de structure des lectines d'animaux vertébrés. Les lectines de poulet et d'anguille comportent 51 résidus identiques sur les 130 positions qui ont été comparées, ce qui correspond à une homologie de 39 %. Les résidus identiques sont répartis sur toute la longueur des protéines, sauf sur le dernier tiers des chaînes, qui présente beaucoup moins de similarités que le reste des polypeptides.

Outre les 51 résidus identiques, 27 positions sont occupées par des résidus homologues, codés par des codons qui ne diffèrent que par un seul nucléotide. Le peptide terminal bloqué sur l'azote se compose d'une séquence Ser-Met N acétylée. L'analyse des peptides résultant du clivage par le CNBr dans un mélange HFBA/acide formique a permis d'assigner au tryptophane la position 70. Cette position 70 assignée au résidu tryptophane est corroborée par le fait que toutes les séquences de lectines représentées à la Fig. 3 présentent la même zone 70-76.

En outre, l'alignement des peptides tel que représenté aux figures 1 et 3 est corroboré par la similarité des zones formant des feuillets plissés β et des profils d'hydropathie, en particulier des séquences de poulet et d'anguille.

La comparaison entre les lectines de poulet et de placenta humain fait apparaître 59 résidus identiques sur 111 positions comparées. En fait, plusieurs séquences de peptides sont identiques dans les deux protéines : les séquences 29-33, 35-40, 45-51, 53-56, 70-16, 78-82, 89-91, 109-111 et 125-127.

La comparaison entre la lectine d'anguille et la lectine de placenta humain fait apparaître 47 résidus identiques sur 111 positions comparées.

La comparaison entre les séquences partielles des quatre lectines d'origine humaine représentées à la Figure 3, fait apparaître des homologies significatives : 50 résidus sur 54 positions comparables (93 % d'homologie) sont identiques dans les lectines de poumon et de placenta humains, alors qu'on ne trouve que 41 % d'homologie entre la lectine de placenta humain et le produit du clone de cDNA d'hépatome humain 1 (42 résidus sur les 103 résidus comparés). Ces résultats indiquent qu'au moins quatre gènes différents codant pour les lectines qui fixent le β-D-galactoside, peuvent être présents dans le génome humain et qu'ils ont une origine commune qui a pour conséquence qu'on retrouve dans les séquences peptidiques des lectines de différentes origines animales, les mêmes déterminants structuraux essentiels, tels, notamment, que le site de fixation du β-D-galactoside qui se situerait, au moins en partie, au niveau des positions 70-76. Cette séquence peptidique contient un résidu tryptophane et deux résidus acide glutamique, respectivement en positions 70, 73 et 76.

### EXEMPLE V - Préparation, par voie de synthèse, d'une séquence d'amino-acides correspondant à au moins une partie d'une lectine fixant le β-D-galactoside

Les peptides séquencés comme décrit à l'Exemple IV qui précède, ont été synthétisés en mettant en oeuvre la méthode en phase solide de MERRIFIELD [cf. J. AM. CHEM. SOC. (1963), 85, 2194].

La synthèse complète de la lectine d'anguille électrique et de la lectine de placenta humain permettra une meilleure approche des propriétés et de la constitution des membranes des lymphocytes et la synthèse de la seconde pourra permettre de disposer d'une source entièrement synthétique pour la préparation d'un agent thérapeutique, immunostimulant et immunosuppresseur, de grande valeur.

### EXEMPLE VI - Préparation d'anticorps polyclonaux contre la lectine placentaire humaine.

### Préparation d'anticorps polyclonaux contre la lectine placentaire humaine.

- on a injecté à des lapins, au temps zéro, 50 µg de lectine placentaire purifiée par chromatographie d'affinité, en adjuvant complet de Freund en injection intra-dermique multi points.
- la deuxième immunisation a eu lieu 15 jours plus tard avec 50 µg de lectine en adjuvant incomplet de Freund en injection sous-cutanée.
- la troisième immunisation a eu lieu 15 jours plus tard avec 50 µg de lectine en adjuvant incomplet de Freund en injection sous-cutanée.
- les saignées ont été effectuées régulièrement toutes les 3 semaines.
- A partir de ces sérums, les fractions immunoglobuliniques ont été purifiées par DEAE puis testées pour déterminer leur activité anticorps par la méthode ELISA et par immunoblot.

### Essai de réponse polyclonale par la méthode ELISA :

- la lectine diluée dans le tampon NaCl β mercaptoéthanol à la concentration de 5 µg/ml, est fixée sur une immunoplaque Nunc comportant 96 puits, à raison de 50 µl par puits pendant 1 h à 30°C.
- 3 lavages en PBS 1 % lait écrémé 0,1 % Tween (PLT).
- Saturation 1 h 30° C.
- 50 µl par puits des différents antisérums et à différentes dilutions (en PLT) sont distribués sur la plaque et incubés 1 h à 30° C.
- 3 lavages en PLT.
- 50 µl par puits de sérum chèvre anti-lapin couplé à la péroxidase dilué au 1/1000 GAR-pox.
- 3 lavages en PBS.
- Révélation par 100 µl de substrat ABTS à 1 % dans du tampon acétique pH 4,7 contenant 0,2 % d'H₂O₂ 30 vol.
- L'absorption est mesurée à une longueur d'onde de 405 nm dans un analyseur Titertek Multiskan.

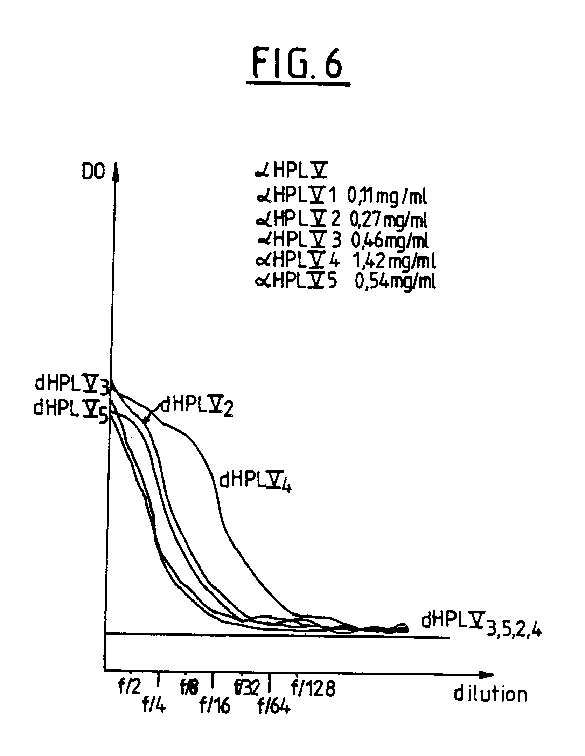

Les résultats obtenus sont représentés à la FiG. 6 annexée.

### Essai de réponse polyclonale mis en évidence par Immunoblot

- On réalise une électrophorèse en SDS-PAGE qui consiste en la migration de la lectine purifiée, en présence de β mercaptoéthanol à raison de 8µg par puits, sur gel d'acrylamide à 15 % contenant 0,1 % de SDS. 250 volts 4 h.
- On transfère ensuite sur nitrocellulose, à 30 volts pendant 16 h.
- On réalise la saturation en PLT pendant 5 h 4°C.
- Des bandes de 1 cm de large sont découpées dans la nitrocellulose.
- On fait incuber des antisérums.
- On lave au PLT.
- GAR-pox 1/1000 (2 ml/bande).
- Lavages PBS Triton 0,1 %.
- La révélation est réalisée au chloronaphtol ;

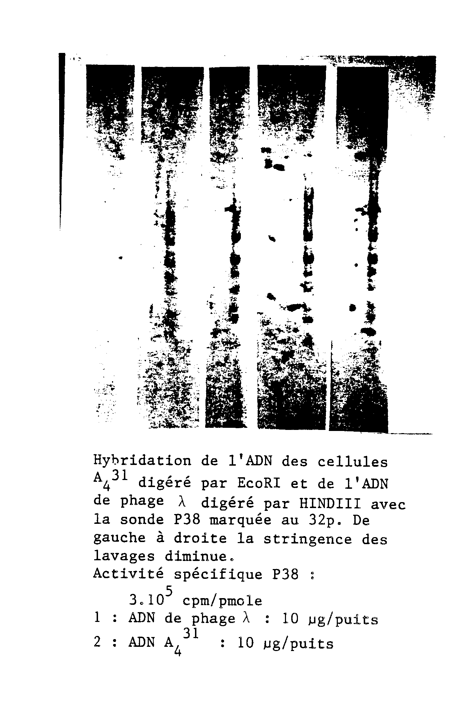

L'immunoblot est représenté à la Fig. 7 annexée qui montre que les antisérums de lapin (fractions IgG purifiées) réagissent avec la lectine : la révélation met en évidence une bande de poids moléculaire 14 KD.
Dans la Fig. 7, les schéma des incubations sont les suivants :
- bande n° 1 : liquide physiologique NaCl 0,15 M, phosphate de sodium 0,01 M, pH 7,4 (PBS).
- bande n° 2 : sérum normal souris au 1/50.
- bande n° 3 : sérum préfusion souris anti-ELH 1/50.
- bande n° 4 : sérum souris anti-ELH 1/50.

### EXEMPLE VII - Préparation d'anticorps monoclonaux anti-lectine placentaire humaine.

### 1. Immunisation

On a immunisé des souris femelles Balb/c en leur injectant une fois par semaine pendant trois semaines consécutives, par voie intra-veineuse, 100 µg de lectine purifiée. Après une période de repos de trois mois, les souris ont eu un rappel par voie intra-veineuse avec la même quantité de lectine trois jours avant le prélèvement des splénocytes pour la fusion.

### 2. Fusion

Des cellules de myélome de souris NS 1 sont utilisées pour la fusion. Elles ont été sélectionnées pour leur sensibilité à l'aminoptérine et cultivées sur le milieu RPMI contenant 10 % de sérum de veau foetal, 2mM de glutamine, 1mM de pyruvate de sodium, 100 UI/ml de pénicilline et 100 µg/ml de streptomycine.

Les splénocytes ont été dispersés par injection du milieu RPMI exempt de sérum dans la rate des souris hyperimmunisées et lavés trois fois dans du milieu RPMI avant fusion.

Les cellules de myélome et les splénocytes ont été ensuite fusionnés dans un rapport de une cellule de myélome pour 10 splénocytes, en présence de 41 % de polyéthylèneglycol d'un poids de 1500 (Merck) selon la méthode de KOHLER et MILSTEIN (Nature, 256, 495-497, 1975), puis lavées dans le milieu RPMI et remises en suspension dans 100 ml du milieu RPMI complet. Les cellules ont ensuite été redistribuées dans des microplaques Nunclon (24 puits par plaque) à raison de 1ml/puits contenant 2,2.10⁵ cellules. Après 24 heures, on a ajouté 1ml/puits de milieu constitué du milieu RPMI complet contenant 0,1 mM d'hypoxanthine, 0,4 M d'aminoptérine et 16 µm de thymidine. Des parties aliquotes de milieu sélectif ont été remplacées les 3e, 6e et 10e jours après la fusion. Après 15 jours, les hybridomes survivants ont été cultivés sur le milieu RPMI complet, complété avec de l'hypoxanthine et de la thymidine et, 15 jours après la fusion, on a recherché la présence d'anticorps anti-lectine placentaire humaine dans les surnageants de culture. Les clones ont été ensuite cultivés progressivement sur le milieu RPMI complet normal.

Les cultures positives ont été clonées par la méthode des dilutions limites selon OI V.T. et HERZENBERG, L.A. 1980 Immunoglobulin-producing hybrid cell lines, in "Selected Methods in Cellular Immunology" (Eds. Mishell, R.B. and Shrigi, S.M., p. 351, Fillman, San Francisco).

Les cellules ont été redistribuées dans les puits d'une microplaque Nunclon, à raison de 0,5 cellule en moyenne par puits, avec 3.10⁵ thymocytes à titre de cellules nutritives. Après 10 jours, les puits contenant des clones uniques ont été sélectionnés et 5 jours plus tard, les surnageants ont été testés pour déterminer la présence d'anticorps antilevures.

Trois clones issus d'une culture primaire positive, ont été sélectionnés et injectés à des souris Balb/c pour obtenir de grandes quantités d'anticorps. A cet effet, des souris femelles agées Balb/c ont été stimulées par une injection intrapéritonéale de 0,3 ml de tétraméthyl-pentadécane. Après 4 jours, 20 millions de cellules hybrides ont été injectées aux souris. Au bout de 15 jours, les fluides d'ascites ont été récoltés et leur activité anti-levure a été testée.

### EXEMPLE VIII - Clonage de sondes oligonucléotidiques.

Le clonage a été réalisé en synthétisant deux sondes oligonucléotidiques basées sur les séquences peptidiques de la lectine placentaire humaine, respectivement entre les résidus 48 et 56 et 70 et 82.

4 espèces moléculaires majeures ont été identifiées dans une banque génomique par la méthode d'hybridation de SOUTHERN et leur clonage réalisé.

De façon plus spécifique, on réalise l'hybridation de l'ADN des cellules A₄³¹ digéré par EcoRI, et de l'ADN de phage λ digéré par HINDIII avec la sonde P38 marquée au ³²P.

L'activité spécifique de la sonde P38 est de 3.10⁵ cpm/pmole.

La figure 7 représente l'hybridation de SOUTHERN réalisée dans les conditions ci-dessus.
1 : ADN de phage λ : 10 µg par puits
2 : ADN A₄³¹ : 10 µg par puits.

### EXEMPLE IX - Applications des anticorps polyclonaux et monoclonaux anti-lectine placentaire humaine.

1. Les anticorps polyclonaux préparés comme décrit à l'Exemple VI ci-dessus, contre la lectine placentaire humaine ont été utilisés pour révéler la présence et la taille de la lectine placentaire dans un mélange de protéines soumis à électrophorèse en gel de polyacrylamide suivie de transfert électrophorétique sur membrane de nitrate de cellulose et d'absorption des anticorps (technique dite "d'immunoblot"), par la technique de l'immunoblot, qui est réalisée comme décrit plus haut à l'Exemple VI.
2. Les anticorps polyclonaux, préparés comme décrit à l'Exemple VI ci-dessus, contre la lectine placentaire humaine, ont été utilisés pour détecter la présence de lectine à la surface de certaines cellules lymphoïdes aussi bien normales que tumorales.
   Les résultats obtenus à ce jour suggèrent que le taux de lectine exprimée à la surface de cellules tumorales varie en fonction de la capacité de ces cellules d'essaimer et de former des métastases.
   Ces anticorps polyclonaux constituent, en conséquence, des agents de détection efficaces de la présence de métastases.
3. Les anticorps polyclonaux ou monoclonaux antilectine constituent, en outre, des agents de diagnostic utiles pour la détermination de la capacité métastasique des tumeurs en utilisant l'une des techniques connues telles qu'immunofluorescence et ELISA, notamment.
4. Les anticorps polyclonaux et monoclonaux antilectine constituent en outre des produits thérapeutiques de valeur, en ce qu'ils peuvent être utilisés en tant que vecteurs pour cibler des toxines ou des enzymes sur des cellules tumorales exprimant la lectine à leur surface.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Séquence d'amino-acides constituant un peptide, caractérisée en ce qu'elle correspond à une partie de la séquence des lectines fixant le β-D-galactoside, en ce que ledit peptide comprend un fragment qui contient un tryptophane ainsi que deux résidus d'acide glutamique, lequel fragment forme au moins en partie le site de fixation du β-D-galactoside et comprend la sequence (a) en amino-acides
Trp-Gly-Thr-Glu-Gln-Arg-Glu (a)
ou une séquence en amino-acides dans laquelle pas plus d'un des amino-acides Thr ou Gln, est remplacé par un autre amino-acide, à condition que ledit peptide ne contienne pas les séquences Gly-Ser-Asn, Met-Glu-Glu, Ala-Gly-Ala ou l'amino-acide Thr immédiatement en amont dudit fragment.

2. Peptide selon la Revendication 1, caractérisé en ce qu'il comprend en outre la séquence :
X₁-X₁-Asn-X₁-Gly (b)
dans laquelle X₁ est un amino-acide hydrophobe choisi dans le groupe qui comprend Val, Ala, Met, Ile et Leu en amont de la séquence (a).

3. Peptide selon la Revendication 2, caractérisé en ce que ladite séquence (b) est située en amont de la séquence (a), à une distance d'environ 35 résidus d'amino-acides.

4. Peptide selon la Revendication 1, caractérisé en ce qu'il inclut en outre, en aval de ladite séquence (a), la séquence en amino-acides suivante :
Ile-Ile-Leu-Pro-Asp-Gly-X₂-X₂-X₂-X₂-Phe-Pro-Asn-Arg-Leu (c)

5. Peptide selon la Revendication 4, caractérisé en ce que ladite séquence (c) est située en aval de la sequence (a), à une distance d'environ 30 résidus d'amino-acides.

6. Peptide selon la revendication 1 ou la revendication 2, caractérisé en ce qu'il comprend, en outre, immédiatement en amont de ladite séquence (a), la séquence en amino-acides (d) suivante :
Asp-Gly-Gly-Ala (d)

7. Peptide selon la revendication 1, caractérisé en ce qu'il inclut, en outre, immédiatement en aval de ladite séquence (a), la séquence en amino-acides (e) suivante :
Ala-Val-Phe-Pro-Phe-Gln-Pro-Gly-Ser-Val-Ala-Glu-Val (e)

8. Peptide selon la revendication 1, caractérisé en ce qu'il comprend un squelette d'amino-acides qui est composé d'au moins les amino-acides suivants, dans les positions suivantes :

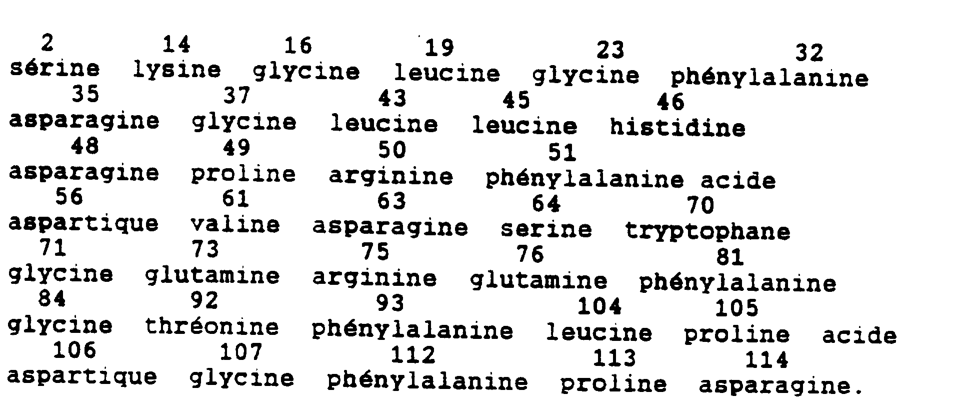

9. Peptide selon la revendication 8, caractérisé en ce que son squelette comprend en outre les amino-acides suivants dans les positions suivantes :

3 5 28 53 54
asparagine valine acide aspartique alanine histidine
55 67 68 72 74 80
glycine glycine glycine thréonine glutamine proline
91 102 103 109
isoleucine isoleucine isoleucine acide glutamique
132 133
isoleucine lysine.

10. Peptide selon la revendication 8, caractérisé en ce que son squelette comprend en outre les amino-acides suivants, dans les positions suivantes :

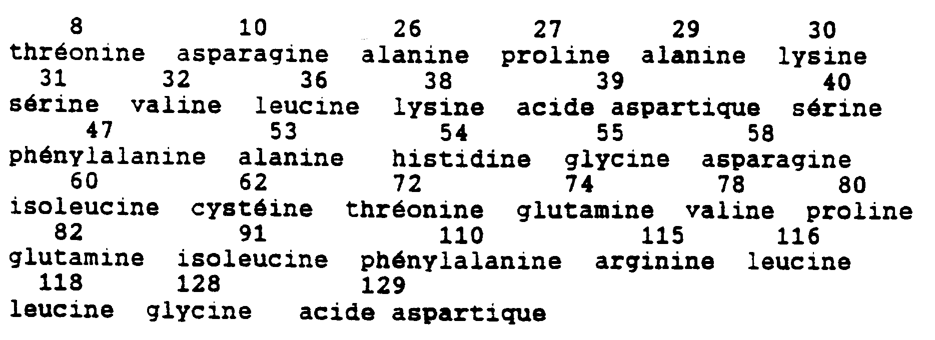

11. Peptide selon la revendication 8, caractérisé en ce que son squelette comprend en outre les amino-acides suivants, dans les positions suivantes :

4 6 18 20 21 22
glycine valine glutamine thréonine valine lysine
52 53 54 55 72
acide aspartique alanine histidine glycine thréonine
74 80 91 96
glutamine proline isoleucine asparagine.

12. Peptide selon la revendication 8, caractérisé en ce que son squelette comprend en outre les amino-acides suivants, dans les positions suivantes :

      28                 67         68         74
acide aspartique  glycine  glycine  glutamine
      79
phénylalanine.

13. Peptide selon la revendication 8, caractérisé en ce que son squelette comprend en outre les amino-acides suivants, dans les positions suivantes :

26 27 29 30 31 33 36
alanine proline alanine lysine sérine valine leucine
38 39 40 74 78
lysine acide aspartique sérine glutamine valine
79 109 110 115 116
phénylalanine glutamine phénylalanine arginine leucine
118 123
leucine tyrosine.

14. Séquence d'amino-acides selon la revendication 1, caractérisée en ce qu'elle comprend au moins les 129 amino-acides suivants, qui sont présents selon la même séquence dans la lectine de l'anguille électrique :

2
(SER MET) ASN GLY VAL VAL ASP GLU ARG MET SER PHE LYS ALA
20 30
GLY GLN ASN LEU THR VAL LYS GLY VAL PRO SER ILE ASP SER THR
40
ASN PHE ALA ILE ASN VAL GLY ASN SER ALA GLU ASP LEU ALA LEU
50 60
HIS ILE ASN PRO ARG PHE ASP ALA HIS GLY ASP GLN GLN ALA VAL
70
VAL VAL ASN SER PHE GLN GLY GLY ASN (TRP) GLY (THR) GLU
80
GLN (ARG) GLU GLY GLY PHE PRO PHE LYS GLN GLY GLU ASP PHE
90 100
LYS ILE GLN ILE THR PHE ASN SER GLU GLU PHE ARG ILE ILE LEU
110
PRO ASP GLY SER GLU ILE HIS PHE PRO ASN ASN ARG TYR MET HIS
120
PHE GLU GLY GLU ALA ARG ILE TYR SER ILE GLU ILE LYS...

15. Séquence d'amino-acides selon la revendication 1, caractérisée en ce qu'elle comprend au moins les amino-acides suivants, qui se présentent selon la même séquence dans la lectine de placenta humain :

 1           4       6         8          10   11
SER (   ) ASN TYR VAL SER (   ) THR (   ) ASN
          18              20  21          23            26      28
                          (   ) ILE GLY GLU VAL ALA PRO ASP
    30  31          34  35  36              40  41  42  43
ALA LYS SER PHE VAL LEU ASN LEU GLY LYS ASP SER ASN ASN LEU
    45  46      48      50  51      53  54  55  56
CYS LEU HIS PHE ASN PRO ARG PHE ASN ALA HIS GLY ASP ALA ASN
    60  61                      67  68      70  71  72
THR ILE VAL LYS ASN SER (   ) ASP GLY GLY ALA TRP GLY THR
    74              78      80  81  82  83  84  85  86  87
GLU GLN ARG GLU ALA VAL PHE PRO PHE GLN PRO GLY SER VAL ALA
 88  89  90  91  92  93  94  95  96  97          100
GLU VAL (   ) ILE THR PHE ASP GLN ALA ASN LEU LEU VAL
101 102 103 104 105     107 108 109 110 111 112 113 114 115
    ILE ILE LEU PRO ASP GLY LEU GLU PHE LYS PHE PRO ASN ARG
116     118 119 120 121     123     125         128     130
LEU ASN LEU GLU ALA ILE ASN LEU MET ALA ALA ASP GLY ASP PHE
131 132 133 134 135
LYS ILE LYS

16. Séquences d'amino-acides caractérisées en ce qu'elles ont été isolées de la séquence d'amino-acides selon la revendication 14 et en ce qu'elles répondent aux formules peptidiques suivantes :

(1)  Asn - Ser - Glu - Glu - Phe - Arg
(2)  Ala - Gly - Gln - Asn - Leu - Thr - Val
(3)  Phe - Asn - Ser - Glu - Glu - Phe - Arg
(4)  Tyr - Met - His - Phe - Glu - Gly - Glu - Ala - Arg
(5)  Glu - Gly - Gly - Phe - Pro
(6)  Ile - Tyr - Ser - Ile - Glu - Ile
(7)  Ile - Tyr - Ser - Ile - Glu - Ile - Lys
(8)  Phe - Asp - Ala - His - Gly - Asp - Gln - Gln - Ala -
     Val - Val - Val - Asn - Ser - Phe - Gln - Gln - Asn
(9)  Ile - Gln - Ile - Thr - Phe - Asn - Ser - Glu - Glu -
     Phe - Arg
(10) Ile - Ile - Leu - Pro - Asp - Gly - Ser - Glu - Ile -
     His - Phe - Pro - Asn - Asn - Arg
(11) Gly - Val - Pro - Ser - Ile - Asp - Ser - Thr - Asn -
     Phe - Ala - Ile - Asn - Val - Gly - Asn - Ser - Ala
     Glu - Asp - Leu - Ala - Leu - His - Ile - Asn - Pro -
     Arg.

17. Séquence d'amino-acides selon l'une quelconque des revendications 1 à 16, caractérisée en ce qu'elle présente une structure en feuillets plissés β et en ce que ces feuillets sont au moins au nombre de 10, dans des positions correspondant à des positions d'amino-acides communs aux lectines de diverses origines.

18. Séquence d'amino-acides selon l'une quelconque des revendications 1 à 17, caractérisée en ce qu'elle contient au moins deux résidus cystéine ou demicystine qui se trouvent en positions 44 et 62.

19. Séquence d'amino-acides selon l'une quelconque des revendications 1 à 18, caractérisée en ce qu'elle comporte un peptide terminal bloqué sur l'azote, qui se compose d'une séquence Ser-Met N-acétylée.

20. Procédé de purification de lectines fixant le β-D-galactoside selon l'une quelconque des revendications 1 à 19, à partir de tissus d'animaux vertébrés et notamment de l'anguille électrique et d'organes humains, tels que le placenta notamment, par homogénéisation et fractionnement des tissus animaux, puis isolement par chromatographie d'affinité à l'aide d'une matrice de lactosylSepharose, lequel procédé est caractérisé en ce que le tampon d'élution utilisé pour isoler les lectines est constitué par une solution saline pH 7,2, tamponnée par du phosphate, additionné de lactose et de 2-mercaptoéthanol.

21. Procédé de détermination des séquences - ou séquençage - d'amino-acides correspondant à une partie de la séquence des lectines fixant le β-D-galactoside selon l'une quelconque des revendications 1 à 19, caractérisé par la mise en oeuvre d'une méthode de dégradation automatique par couplage de phénylisothiocyanate avec l'azote terminal du peptide à séquencer, clivage dudit résidu amino-terminal par cyclisation en milieu acide, et conversion du dérivé de thiazolinom formé, en un dérivé de phénylthiohydantoïne, laquelle méthode de dégradation est réalisée en présence de polybrène.

22. Procédé d'obtention par voie de synthèse de séquences d'amino-acides correspondant à une partie de la séquence de lectines fixant le β-D-galactoside, caractérisé en ce que ladite synthèse est réalisée en mettant en oeuvre une méthode dérivée de la méthode de BERGMAN et ZERVAS et utilisant des groupes protecteurs pour protéger les fonctions réactives des amino-acides à coupler, et des méthodes de couplage appropriées pour coupler entre eux les amino-acides protégés, pour former les séquences recherchées selon l'une quelconque des revendications 1 à 19.

23. Anticorps polyclonaux anti-lectine placentaire humaine, caractérisés en ce qu'ils sont essentiellement constitués par des sérums obtenus à partir de sang d'animaux immunisés par injection de lectine selon l'une quelconque des revendications 1 à 15 et 17 à 19, purifiée selon la revendication 20.

24. Anticorps monoclonaux anti-lectine placentaire humaine, caractérisés ence qu'ils sont constitués par des clones isolés à partir d'hybridomes obtenus par fusion de cellules tumorales appropriées, SP 2-0 ou NS-1 notamment, avec des splénocytes de souris immunisées contre la lectine de placenta humain, selon l'une quelconque des revendications 1 à 15 et 17 à 19, convenablement purifiée selon la revendication 20.

25. Agent de diagnostic d'affections tumorales, caractérisé en ce qu'il comprend des anticorps polyclonaux selon la revendication 23 et/ou des anticorps monoclonaux selon la revendication 24.

26. Agent thérapeutique caractérisé en ce qu'il comprend une lectine selon la revendication 1 ou la revendication 7, éventuellement associée à au moins un véhicule pharmaceutiquement acceptable.

27. Agent thérapeutique caractérisé en ce qu'il comprend des anticorps polyclonaux selon la revendication 23 et/ou des anticorps monoclonaux selon la revendication 24, éventuellement associés à des toxines et/ou des enzymes.

## Claims

1. Amino acid sequence constituting a peptide, characterized in that it corresponds to part of the sequence of lectins binding β-D-galactoside, and in that the said peptide comprises a fragment which contains a tryptophan as well as two glutamic acid residues, which fragment forms at least in part the β-D-galactoside-binding site and comprises the amino acid sequence (a)
Trp-Gly-Thr-Glu-Gln-Arg-Glu (a)
or an amino acid sequence in which not more than one of the amino acids Thr or Gln has been replaced by another amino acid, with the proviso that the said peptide does not contain the sequences Gly-Ser-Asn, Met-Glu-Glu, Ala-Gly-Ala or the amino acid Thr immediately upstream of the said fragment.

2. Peptide according to Claim 1, characterized in that it additionally comprises the sequence:
X₁-X₁-Asn-X₁-Gly (b)
in which X₁ is a hydrophobic amino acid chosen from the group comprising Val, Ala, Met, Ile and Leu upstream of the sequence (a).

3. Peptide according to Claim 2, characterized in that the said sequence (b) is located upstream of the sequence (a) at a distance of about 35 amino acid residues.

4. Peptide according to Claim 1, characterized in that it additionally includes, downstream of the said sequence (a), the following amino acid sequence:
Ile-Ile-Leu-Pro-Asp-Gly-X₂-X₂-X₂-X₂-Phe-Pro-Asn-Arg-Leu (c)

5. Peptide according to Claim 4, characterized in that the said sequence (c) is located downstream of the sequence (a) at a distance of about 30 amino acid residues.

6. Peptide according to Claim 1 or Claim 2, characterized in that it additionally comprises, immediately upstream of the said sequence (a), the following amino acid sequence (d):
Asp-Gly-Gly-Ala (d)

7. Peptide according to Claim 1, characterized in that it additionally includes, immediately downstream of the said sequence (a), the following amino acid sequence (e):
Ala-Val-Phe-Pro-Phe-Gln-Pro-Gly-Ser-Val-Ala-Glu-Val (e)

8. Peptide according to Claim 1, characterized in that it comprises an amino acid skeleton which is composed of at least the following amino acids, in the following positions:

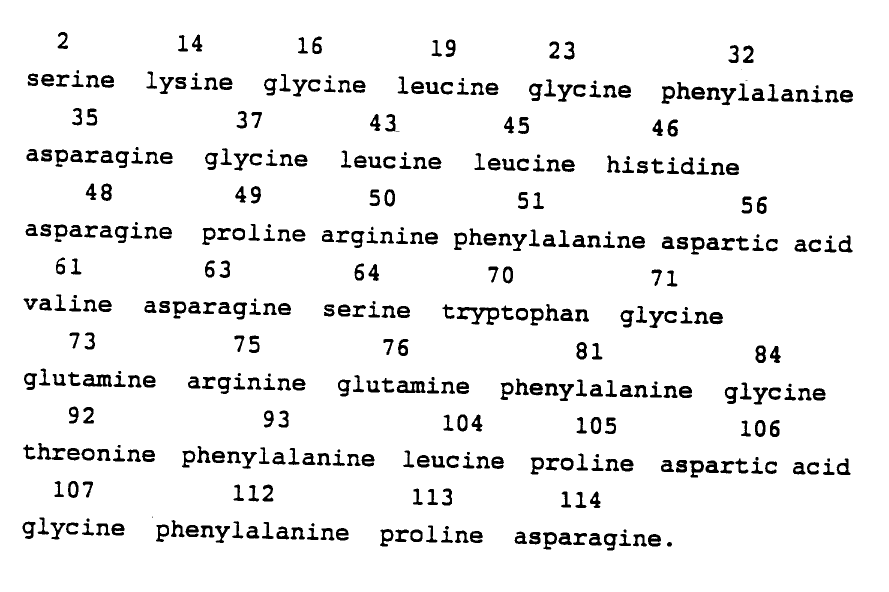

9. Peptide according to Claim 8, characterized in that its skeleton additionally comprises the following amino acids in the following positions:

3 5 28 53 54
asparagine valine aspartic acid alanine histidine
55 67 68 72 74 80
glycine glycine glycine threonine glutamine proline
91 102 103 109
isoleucine isoleucine isoleucine glutamic acid
132 133
isoleucine lysine.

10. Peptide according to Claim 8, characterized in that its skeleton additionally comprises the following amino acids, in the following positions:

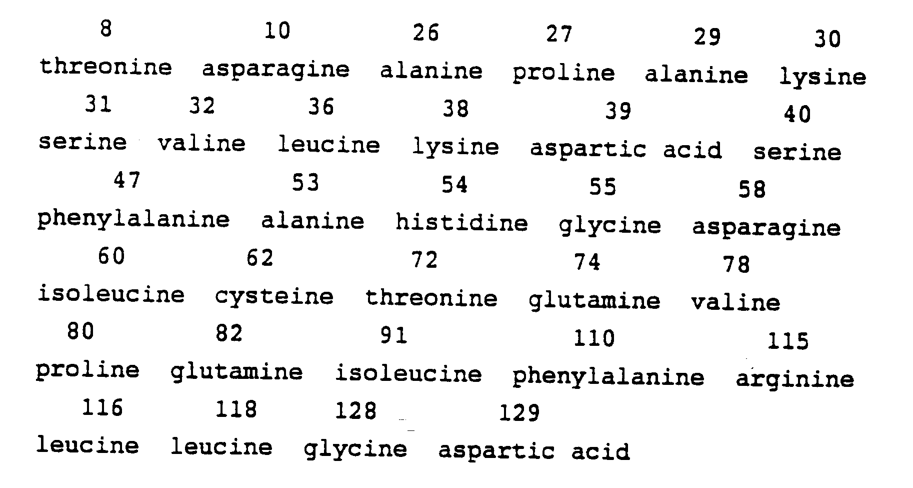

11. Peptide according to Claim 8, characterized in that its skeleton additionally comprises the following amino acids, in the following positions:

4 6 18 20 21 22
glycine valine glutamine threonine valine lysine
52 53 54 55 72
aspartic acid alanine histidine glycine threonine
74 80 91 96
glutamine proline isoleucine asparagine.

12. Peptide according to Claim 8, characterized in that its skeleton additionally comprises the following amino acids, in the following positions:

28 67 68 74
aspartic acid glycine glycine glutamine
79
phenylalanine.

13. Peptide according to Claim 8, characterized in that its skeleton additionally comprises the following amino acids, in the following positions:

26 27 29 30 31 33
alanine proline alanine lysine serine valine
36 38 39 40 74 78
leucine lysine aspartic acid serine glutamine valine
79 109 110 115
phenylalanine glutamine phenylalanine arginine
116 118 123
leucine leucine tyrosine.

14. Amino acid sequence according to Claim 1, characterized in that it comprises at least the following 129 amino acids, which are present according to the same sequence in the lectin of the electric eel:

2
                                                    10
(SER MET) ASN GLY VAL VAL ASP GLU ARG MET SER
                                        20
PHE LYS ALA GLY GLN ASN LEU THR VAL LYS GLY
                                  30
VAL PRO SER ILE ASP SER THR ASN PHE ALA ILE
                            40
ASN VAL GLY ASN SER ALA GLU ASP LEU ALA LEU
                      50
HIS ILE ASN PRO ARG PHE ASP ALA HIS GLY ASP
                60
GLN GLN ALA VAL VAL VAL ASN SER PHE GLN GLY
          70
GLY ASN (TRP) GLY (THR) GLU GLN (ARG) GLU GLY GLY
    80
PHE PRO PHE LYS GLN GLY GLU ASP PHE LYS ILE
90                                                  100
GLN ILE THR PHE ASN SER GLU GLU PHE ARG ILE
                                              110
ILE LEU PRO ASP GLY SER GLU ILE HIS PHE PRO
                                        120
ASN ASN ARG TYR MET HIS PHE GLU GLY GLU ALA
ARG ILE TYR SER ILE GLU ILE LYS...

15. Amino acid sequence according to Claim 1, characterized in that it comprises at least the following amino acids, which appear according to the same sequence in the lectin of human placenta:

1
SER ( ) ASN TYR VAL SER ( ) THR ( ) ASN
4 6 8 10 11
18 20 21 23
( ) ILE GLY GLU VAL

26 28 30 31 34 35 36
ALA PRO ASP ALA LYS SER PHE VAL LEU ASN LEU
40 41 42 43 45 46
GLY LYS ASP SER ASN ASN LEU CYS LEU HIS PHE
48 50 51 53 54 55 56
ASN PRO ARG PHE ASN ALA HIS GLY ASP ALA ASN
60 61 67 68
THR ILE VAL LYS ASN SER ( ) ASP GLY GLY ALA
70 71 72 74 78 80
TRP GLY THR GLU GLN ARG GLU ALA VAL PHE PRO
81 82 83 84 85 86 87 88 89 91
PHE GLN PRO GLY SER VAL ALA GLU VAL ( 90 ) ILE
92 93 94 95 96 97 100 101 102
THR PHE ASP GLN ALA ASN LEU LEU VAL ILE
103 104 105 107 108 109 110 111 112 113
ILE LEU PRO ASP GLY LEU GLU PHE LYS PHE PRO
114 115 116 118 119 120 121 123 125
ASN ARG LEU ASN LEU GLU ALA ILE ASN LEU MET ALA
128 130 131 132 133 134 135
ALA ASP GLY ASP PHE LYS ILE LYS

16. Amino acid sequences characterized in that they have been isolated from the amino acid sequence according to Claim 14 and in that they correspond to the following peptide formulae:

(1) Asn - Ser - Glu - Glu - Phe - Arg
(2) Ala - Gly - Gln - Asn - Leu - Thr - Val
(3) Phe - Asn - Ser - Glu - Glu - Phe - Arg
(4) Tyr - Met - His - Phe - Glu - Gly - Glu - Ala - Arg
(5) Glu - Gly - Gly - Phe - Pro
(6) Ile - Tyr - Ser - Ile - Glu - Ile
(7) Ile - Tyr - Ser - Ile - Glu - Ile - Lys
(8) Phe - Asp - Ala - His - Gly - Asp - Gln - Gln - Ala -
Val - Val - Val - Asn - Ser - Phe - Gln - Gln - Asn
(9) Ile - Gln - Ile - Thr - Phe - Asn - Ser - Glu - Glu -

Phe - Arg
(10) Ile - Ile - Leu - Pro - Asp - Gly - Ser - Glu - Ile -
His - Phe - Pro - Asn - Asn - Arg
(11) Gly - Val - Pro - Ser - Ile - Asp - Ser - Tlr - Asn -
Phe - Ala - Ile - Asn - Val - Gly - Asn - Ser - Ala
Glu - Asp - Leu - Ala - Leu - His - Ile - Asn - Pro -
Arg.

17. Amino acid sequence according to any one of Claims 1 to 16, characterized in that it has a β-pleated sheet structure and in that these sheets are at least 10 in number, in positions of amino acids common to lectins of diverse origin.

18. Amino acid sequence according to any one of Claims 1 to 17, characterized in that it contains at least two cysteine or half-cystine residues which are in positions 44 and 62.

19. Amino acid sequence according to any one of Claims 1 to 18, characterized in that it contains a terminal peptide blocked on the nitrogen, which is composed of an N-acetylated Ser-Met sequence.

20. Process for the purification of lectins binding β-D-galactoside according to any one of Claims 1 to 19, starting from tissues of vertebrate animals and in particular the electric eel and of human organs, such as, in particular, the placenta, by homogenization and fractionation of the animal tissues, followed by isolation by affinity chromatography with the aid of a matrix of lactosyl-Sepharose, which process is characterized in that the elution buffer used to isolate the lectins consists of a phosphate-buffered saline solution of pH 7.2 to which lactose and 2-mercaptoethanol have been added.

21. Process for the determination of the sequences - or sequencing - of amino acids corresponding to part of the sequence of lectins binding β-D-galactoside according to any one of Claims 1 to 19, characterized by the use of an automatic degradation method by coupling phenyl isothiocyanate with the terminal nitrogen of the peptide to be sequenced, cleavage of the said amino-terminal residue by cyclization in an acid medium and conversion of the thiazolinone derivative formed to a phenylthiohydantoin derivative, which degradation method is carried out in the presence of Polybrene.

22. Process for obtaining, by synthesis, amino acid sequences corresponding to part of the sequence of lectins binding β-D-galactoside, characterized in that the said synthesis is carried out using a method derived from the method of BERGMAN and ZERVAS and using protective groups to protect the reactive functions of the amino acids to be coupled, and appropriate coupling methods for coupling the protected amino acids with one another in order to form the sequences sought according to any of Claims 1 to 19.

23. Anti-human placental lectin polyclonal antibodies, characterized in that they essentially consist of sera obtained from blood of animals immunized by injection of lectin according to any one of Claims 1 to 15 and 17 to 19, purified according to Claim 20.

24. Anti-human placental lectin monoclonal antibodies, characterized in that they consist of clones isolated from hybridomas obtained by fusion of appropriate tumour cells, in particular SP 2-0 or NS-1, with spleen cells of mice immunized against the lectin of human placenta, according to any one of Claims 1 to 15 and 17 to 19, appropriately purified according to Claim 20.

25. Agent for the diagnosis of tumour conditions, characterized in that it comprises polyclonal antibodies according to Claim 23 and/or monoclonal antibodies according to Claim 24.

26. Therapeutic agent characterized in that it comprises a lectin according to Claim 1 or Claim 7, optionally in combination with at least one pharmaceutically acceptable excipient.

27. Therapeutic agent characterized in that it comprises polyclonal antibodies according to Claim 23 and/or monoclonal antibodies according to Claim 24, optionally in combination with toxins and/or enzymes.

## Patentansprüche

1. Ein Peptid bildende Aminosäuresequenz, dadurch **gekennzeichnet,** daß sie einem Teil der Sequenz der Lectine, die das β-D-Galactosid binden, entspricht, wobei das Peptid ein Fragment umfaßt, das einen Tryptophanrest sowie zwei Glutaminsäurereste enthält, wobei das Fragment mindestens teilweise die Bindungsstelle des β-D-Galactosids bildet und die Aminosäuresequenz (a)
Trp-Gly-Thr-Glu-Gln-Arg-Glu (a)
oder eine Aminosäuresequenz, worin nicht mehr als eine der Aminosäuren Thr oder Gln durch eine andere Aminosäure ersetzt ist, umfaßt mit der Maßgabe, daß das Peptid die Sequenzen Gly-Ser-Asn, Met-Glu-Glu, Ala-Gly-Ala oder die Aminosäure Thr unmittelbar stromaufwärts des Fragments nicht enthält.

2. Peptid nach Anspruch 1, dadurch **gekennzeichnet,** daß es weiterhin die Sequenz
X₁-X₁-Asn-X₁-Gly (b)
umfaßt, worin X₁ für eine hydrophobe Aminosäure, ausgewählt aus der Gruppe Val, Ala, Met, Ile und Leu, stromaufwärts der Sequenz (a) ist.

3. Peptid nach Anspruch 2, dadurch **gekennzeichnet,** daß die Sequenz (b) stromaufwärts der Sequenz (a) in einer Entfernung von etwa 35 Aminosäureresten sich befindet.

4. Peptid nach Anspruch 1, dadurch **gekennzeichnet,** daß es weiterhin stromabwärts der Sequenz (a) folgende Aminosäuresequenz
Ile-Ile-Leu-Pro-Asp-Gly-X₂-X₂-X₂-X₂-Phe-Pro-Asn-Arg-Leu (c)
umfaßt.

5. Peptid nach Anspruch 4, dadurch **gekennzeichnet,** daß die Sequenz (c) stromabwärts der Sequenz (a) in einer Entfernung von etwa 30 Aminosäureresten sich befindet.

6. Peptid nach Anspruch 1 oder 2, dadurch **gekennzeichnet,** daß es weiterhin unmittelbar stromaufwärts der Sequenz (a) die folgende Aminosäuresequenz (d)
Asp-Gly-Gly-Ala (d)
umfaßt.

7. Peptid nach Anspruch 1, dadurch **gekennzeichnet,** daß es weiterhin unmittelbar stromabwärts der Sequenz (a) die folgende Aminosäuresequenz (e)
Ala-Val-Phe-Pro-Phe-Gln-Pro-Gly-Ser-Val-Ala-Glu-Val (e)
umfaßt.

8. Peptid nach Anspruch 1, dadurch **gekennzeichnet,** daß es ein Aminosäureskelett umfaßt, das aus mindestens den folgenden Aminosäuren in den folgenden Positionen besteht:

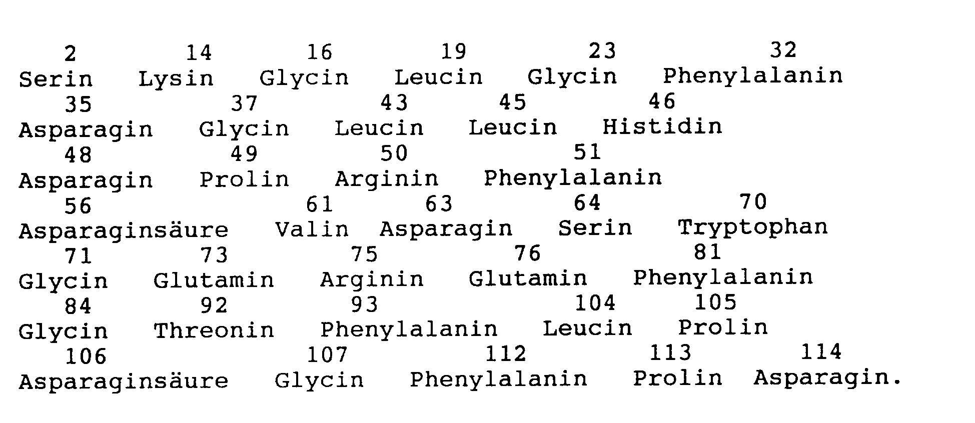

9. Peptid nach Anspruch 8, dadurch **gekennzeichnet,** daß sein Skelett weiterhin die folgenden Aminosäuren in den folgenden Positionen umfaßt:

3 5 28 53 54
Asparagin Valin Asparaginsäure Alanin Histidin
55 67 68 72 74 80
Glycin Glycin Glycin Threonin Glutamin Prolin
91 102 103 109
Isoleucin Isoleucin Isoleucin Glutaminsäure
132 133
Isoleucin Lysin.

10. Peptid nach Anspruch 8, dadurch **gekennzeichnet,** daß sein Skelett weiterhin die folgenden Aminosäuren in den folgenden Positionen umfaßt:

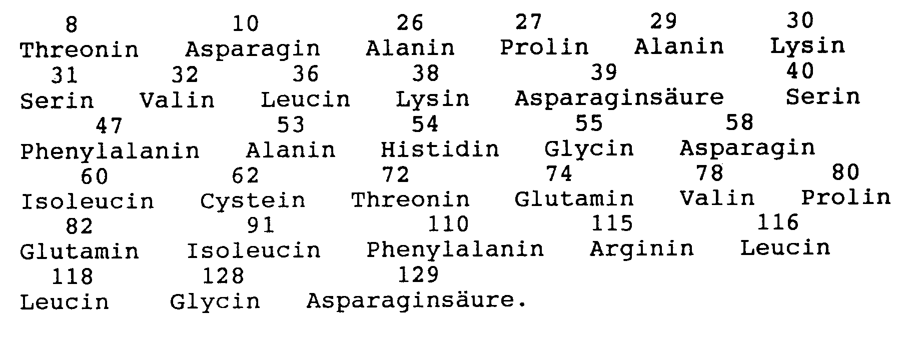

11. Peptid nach Anspruch 8, dadurch **gekennzeichnet,** daß sein Skelett weiterhin die folgenden Aminosäuren in den folgenden Positionen umfaßt:

4 6 18 20 21 22
Glycin Valin Glutamin Threonin Valin Lysin
52 53 54 55 72
Asparaginsäure Alanin Histidin Glycin Threonin
74 80 91 96
Glutamin Prolin Isoleucin Asparagin.

12. Peptid nach Anspruch 8, dadurch **gekennzeichnet,** daß sein Skelett weiterhin die folgenden Aminosäuren in den folgenden Positionen umfaßt:

28 67 68 74
Asparaginsäure Glycin Glycin Glutamin
79
Phenylalanin.

13. Peptid nach Anspruch 8, dadurch **gekennzeichnet,** daß sein Skelett weiterhin die folgenden Aminosäuren in den folgenden Positionen umfaßt:

26        27        29        30       31       33        36
Alanin    Prolin    Alanin    Lysin    Serin    Valin     Leucin
38              39                40       74          78
Lysin    Asparaginsäure    Serin    Glutamin    Valin
79                  109              110                115       116
Phenylalanin    Glutamin    Phenylalanin    Arginin    Leucin
118       123
Leucin    Tyrosin.

14. Aminosäuresequenz nach Anspruch 1, dadurch **gekennzeichnet,** daß sie mindestens die folgenden 129 Aminosäuren umfaßt, die in der gleichen Sequenz des Lectins des Zitteraals vorhanden sind:

2                                   10
(SER MET) ASN GLY VAL VAL ASP GLU ARG MET SER PHE LYS ALA
                  20                                    30
GLY GLN ASN LEU THR VAL LYS GLY VAL PRO SER ILE ASP SER THR
                                            40
ASN PHE ALA ILE ASN VAL GLY ASN SER ALA GLU ASP LEU ALA LEU
                  50                                    60
HIS ILE ASN PRO ARG PHE ASP ALA HIS GLY ASP GLN GLN ALA VAL
                                             70
VAL VAL ASN SER PHE GLN GLY GLY ASN (TRP) GLY (THR) GLU
                              80
GLN (ARG) GLU GLY GLY PHE PRO PHE LYS GLN GLY GLU ASP PHE
          90                                        100
LYS ILE GLN ILE THR PHE ASN SER GLU GLU PHE ARG ILE ILE LEU
                               110
PRO ASP GLY SER GLU ILE HIS PHE PRO ASN ASN ARG TYR MET HIS
         120
PHE GLU GLY GLU ALA ARG ILE TYR SER ILE GLU ILE LYS...

15. Aminosäuresequenz nach Anspruch 1, dadurch **gekennzeichnet,** daß sie mindestens die folgenden Aminosäuren umfaßt, die in der gleichen Sequenz des Lectins der menschlichen Plazenta vorhanden sind:

1                4        6          8          10   11
SER (   ) ASN TYR VAL SER (   ) THR (    ) ASN
          18                20  21          23              26      28
                            (   ) ILE GLY GLU VAL ALA PRO ASP
     30  31             34  35  36                  40  41  42  43
ALA LYS SER PHE VAL LEU ASN LEU GLY LYS ASP SER ASN ASN LEU
     45  46      48      50  51      53  54  55  56
CYS LEU HIS PHE ASN PRO ARG PHE ASN ALA HIS GLY ASP ALA ASN
     60  61                          67  68      70  71  72
THR ILE VAL LYS ASN SER (   ) ASP GLY GLY ALA TRP GLY THR
     74              78      80  81  82  83  84  85  86  87
GLU GLN ARG GLU ALA VAL PHE PRO PHE GLN PRO GLY SER VAL ALA
 88  89  90   91  92  93  94  95  96  97          100
GLU VAL (   ) ILE THR PHE ASP GLN ALA ASN LEU LEU VAL
101 102 103 104 105     107 108 109 110 111 112 113 114 115
    ILE ILE LEU PRO ASP GLY LEU GLU PHE LYS PHE PRO ASN ARG
116     118 119 120 121     123     125         128     130
LEU ASN LEU GLU ALA ILE ASN LEU MET ALA ALA ASP GLY ASP PHE
131 132 133 134 135
LYS ILE LYS

16. Aminosäuresequenzen, dadurch **gekennzeichnet,** daß sie aus der Aminosäuresequenz nach Anspruch 14 isoliert worden sind und daß sie den folgenden Peptidformeln entsprechen:

(1)  Asn - Ser - Glu - Glu - Phe - Arg
(2)  Ala - Gly - Gln - Asn - Leu - Thr - Val
(3)  Phe - Asn - Ser - Glu - Glu - Phe - Arg
(4)  Tyr - Met - His - Phe - Glu - Gly - Glu - Ala - Arg
(5)  Glu - Gly - Gly - Phe - Pro
(6)  Ile - Tyr - Ser - Ile - Glu - Ile
(7)  Ile - Tyr - Ser - Ile - Glu - Ile - Lys
(8)  Phe - Asp - Ala - His - Gly - Asp - Gln - Gln - Ala -
     Val - Val - Val - Asn - Ser - Phe - Gln - Gln - Asn
(9)  Ile - Gln - Ile - Thr - Phe - Asn - Ser - Glu - Glu -
     Phe - Arg
(10) Ile - Ile - Leu - Pro - Asp - Gly - Ser - Glu - Ile -
     His - Phe - Pro - Asn - Asn - Arg
(11) Gly - Val - Pro - Ser - Ile - Asp - Ser - Thr - Asn -
     Phe - Ala - Ile - Asn - Val - Gly - Asn - Ser - Ala
     Glu - Asp - Leu - Ala - Leu - His - Ile - Asn - Pro -
     Arg.

17. Aminosäuresequenz nach einem der Ansprüche 1 bis 16, dadurch **gekennzeichnet,** daß sie eine β-Faltblattstruktur besitzt und daß die Faltblätter mindestens in einer Anzahl von 10 an Positionen, die Aminosäurepositionen entsprechen, die den Lectinen verschiedener Ursprünge gemeinsam sind, vorhanden sind.

18. Aminosäuresequenz nach einem der Ansprüche 1 bis 17, dadurch **gekennzeichnet,** daß sie mindestens zwei Cystein- oder Semicystinreste enthält, die sich an den Positionen 44 und 62 befinden.

19. Aminosäuresequenz nach einem der Ansprüche 1 bis 18, dadurch **gekennzeichnet,** daß sie ein endständiges, am Stickstoff blockiertes Peptid enthält, das aus einer N-Acetyl-Ser-Met-Sequenz besteht.

20. Verfahren zur Reinigung von Lectinen, die β-D-Galactosid binden, nach einem der Ansprüche 1 bis 19 aus Geweben von Vertebraten und insbesondere des Zitteraals und aus menschlichen Organen, wie insbesondere der Plazenta, durch Homogenisierung und Fraktionierung der tierischen Gewebe und anschließende affinitätschromatographische Isolierung mit Hilfe einer Lactosyl-Sepharose-Matrix, dadurch **gekennzeichnet,** daß der Elutionspuffer, der zur Isolierung der Lectine verwendet wird, aus einer Salzlösung mit einem pH von 7,2, gepuffert durch Phosphat, unter Zusatz von Lactose und 2-Mercaptoethanol, besteht.

21. Verfahren zur Bestimmung der Sequenzen - oder der Sequenzabfolge - an Aminosäuren, entsprechend einem Teil der Sequenz der Lectine, die das β-D-Galactosid fixieren, nach einem der Ansprüche 1 bis 19, dadurch **gekennzeichnet,** daß ein automatisches Abbauverfahren durch Phenylisothiocyanat-Kupplung mit dem endständigen Stickstoff des zu sequenzierenden Peptids, Spaltung des aminoterminalen Rests durch Cyclisierung in saurem Milieu und Umwandlung des gebildeten Thiazolinonderivats in ein Phenylthiohydantoinderivat verwendet wird, wobei das Abbauverfahren in Gegenwart von Polybren durchgeführt wird.

22. Verfahren zur Herstellung mittels Synthese von Aminosäuresequenzen, entsprechend einem Teil der Sequenz der Lectine, die das β-D-Galactosid binden, dadurch **gekennzeichnet,** daß die Synthese durch Durchführen eines Verfahrens, das sich von dem BERGMAN-ZERVAS-Verfahren ableitet, unter Verwendung von Schutzgruppen zum Schutz der reaktiven Funktionen der zu kuppelnden Aminosäuren und von geeigneten Verfahren zur Kupplung, um die geschützten Aminosäuren untereinander zu verbinden, durchgeführt wird, um die nach einem der Ansprüche 1 bis 19 gewünschten Sequenzen zu bilden.

23. Polyclonale Anti-menschliche-Plazenta-Lectin-Antikörper, dadurch **gekennzeichnet,** daß sie im wesentlichen aus Seren bestehen, die aus dem Blut von Tieren, die durch Injektion mit einem Lectin nach einem der Ansprüche 1 bis 15 und 17 bis 19, das nach Anspruch 20 gereinigt wurde, immunisiert worden sind, erhalten worden sind.

24. Monoclonale Anti-menschliche-Plazenta-Lectin-Antikörper, dadurch **gekennzeichnet,** daß sie aus Clonen bestehen, die von Hybridomen isoliert worden sind, die durch Fusion von geeigneten Tumorzellen, insbesondere SP 2-0 oder NS-1, mit Milzzellen von Mäusen, die gegen das Lectin der menschlichen Plazenta nach einem der Ansprüche 1 bis 15 und 17 bis 19, das geeigneterweise nach Anspruch 20 gereinigt worden ist, immunisiert worden sind, erhalten worden sind.

25. Mittel zur Diagnose von Tumorbefall, dadurch **gekennzeichnet,** daß es polyclonale Antikörper nach Anspruch 23 und/oder monoclonale Antikörper nach Anspruch 24 umfaßt.

26. Therapeutisches Mittel, dadurch **gekennzeichnet,** daß es ein Lectin nach Anspruch 1 oder 7 gegebenenfalls zusammen mit mindestens einem pharmazeutisch annehmbaren Träger enthält.

27. Therapeutisches Mittel, dadurch **gekennzeichnet,** daß es polyclonale Antikörper nach Anspruch 23 und/oder monoclonale Antikörper nach Anspruch 24 gegebenenfalls zusammen mit Toxinen und/oder Enzymen enthält.
